# EUROPÄISCHE PATENTSCHRIFT

(11) **EP 0 111 711 B1**
(45) Veröffentlichungstag und Bekanntmachung des Hinweises auf die Patenterteilung: **18.03.1987**
(21) Anmeldenummer: 83110995.4
(22) Anmeldetag: 04.11.1983
(51) Int. Cl.: C07D 405/06, C07D 405/14, A01N 43/653, A01N 43/50, C07D 407/06, C07D 317/26, C07D 407/04, C07D 319/06

(54) **Heterocyclisch substituierte Hydroxyalkyl-azolyl-Derivate**
Heterocyclically substituted hydroxyalkyl-azolyl derivatives
Dérivés hydroxyalkyl-azolyles substitués par des hétérocycles

(30) Priorität: 15.11.1982 DE 3242252
(43) Veröffentlichungstag der Anmeldung: 27.06.1984
(60) Teilanmeldung: 85113427.0
(73) Patentinhaber: BAYER AG, 51368 Leverkusen (DE)
(72) Erfinder: Krämer, Wolfgang, Dr., D-5600 Wuppertal 1 (DE); Büchel, Karl Heinz, Prof.Dr., D-5093 Burscheid (DE); Holmwood, Graham, Dr., D-5600 Wuppertal 11 (DE); Regel, Erik, Dipl.-Ing., D-5600 Wuppertal 1 (DE); Reinecke, Paul, Dr., D-5090 Leverkusen 3 (DE); Brandes, Wilhelm, Dr., D-5653 Leichlingen 1 (DE)

## Beschreibung

Die vorliegende Erfindung betrifft neue heterocyclisch substituierte Hydroxyalkyl-azolyl-Derivate, mehrere Verfahren zu ihrer Herstellung und ihre Verwendung als Fungizide.

Es ist bereits bekannt geworden, daß bestimmte Hydroxyethyl-azolyl-Derivate, wie z.B. 3,3-Dimethyl-1-phenoxy-2-(1,2,4-triazol-1-yl-methyl)-2-butanol bzw. 1-(2-Chlor-4-fluorphenoxy)-3,3-dimethyl-1-(1,2,4-triazol-1-yl-methyl)-2-butanol oder 1-(4-Chlorphenoxy)-2-(2,4-dichlorphenyl)-3-(imidazol-1-yl)-2-propanol, fungizide Eigenschaften aufweisen (vgl. DE-A 30 18 866 bzw. EP-A 0 040 345). Die Wirksamkeit dieser Verbindungen ist jedoch, insbesondere bei niedrigen Aufwandmengen und -konzentrationen, nicht immer ganz befriedigend. Außerdem enthält keine dieser Verbindungen einen Dioxolan-2-yl- oder 1,3-Dioxanyl-Rest.

In der EP-A 0 078 594 werden Substanzen beschrieben, in denen ein Dioxolan-2-yl-Rest vorhanden sein kann. In keinem Fall ist dieser Heterocyclus aber über eine -C(CH₃)₂-Gruppe mit dem verbleibenden Molekülteil verbunden.

Aus der EP-A 0 085 333 und der EP-A 0 084 834 sind fungizid wirksame Hydroxyalkyl-azolyl-Derivate bekannt, in denen einer der Substituenten am Carbinol-Kohlenstoffatom für gegebenenfalls substituiertes Alkyl stehen kann. Es werden jedoch keine Verbindungen erwähnt, in denen der betreffende Rest einen über ein verzweigtes Alkyl verknüpften Dioxolan-2-yl- oder 1,3-Dioxanyl-Rest bedeutet.

In der EP-A 0 062 236 werden Stoffe aufgeführt, die einen Dioxanyl-Rest aufweisen, welcher aber nicht über eine -C(CH₃)₂-Einheit mit dem zentralen Kohlenstoffatom verbunden ist. Außerdem ist auch der Azolyl-Rest nicht über -CH₂- mit dem Carbinol-Kohlenstoffatom verknüpft.

Es wurden neue, heterocyclisch substituierte Hydroxyalkylazolyl-Derivate der allgemeinen Formel 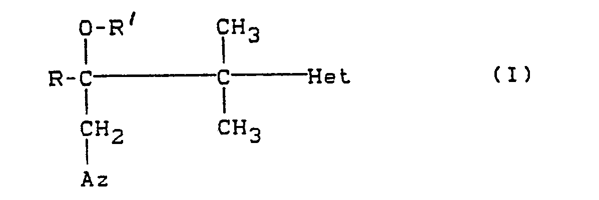 in welcher
Az für 1,2,4-Triazol-1-yl, 1,2,4-Triazol-4-yl oder Imidazol-1-yl steht,
Het für jeweils gegebenenfalls einfach oder mehrfach, gleich oder verschieden substituiertes Dioxolan-2-yl oder 1,3-Dioxanyl steht, wobei als Substituenten zu nennen sind: Alkyl mit 1 bis 4 Kohlenstoffatomen, sowie jeweils gegebenenfalls einfach oder mehrfach, gleich oder verschieden substituiertes Phenyl und Phenoxyalkyl mit 1 bis 4 Kohlenstoffatomen im Alkylteil, wobei als Phenylsubstituenten zu nennen sind: Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen;
R für geradkettiges oder verzweigtes Alkyl mit 1 bis 7 Kohlenstoffatomen steht, ferner für jeweils gegebenenfalls einfach oder mehrfach, gleich oder verschieden substituiertes Phenyl, Phenylalkyl mit 1 bis 4 Kohlenstoffatomen im Alkylteil, Phenoxy- und Phenylthioalkyl mit jeweils 1 bis 4 Kohlenstoffatomen im Alkylteil oder für Phenylethenyl steht, wobei als Substituenten an den Phenyl-Gruppen genannt werden: Halogen, Alkyl mit 1 bis 4 Kohlenstoffatomen, Alkoxy und Alkylthio mit jeweils 1 bis 2 Kohlenstoffatomen, Nitro, Halogenalkyl und Halogenalkoxy sowie Halogenalkylthio mit jeweils 1 bis 2 Kohlenstoffatomen und 1 bis 5 gleichen oder verschiedenen Halogenatomen, Hydroximinoalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und Alkyl mit 1 bis 2 Kohlenstoffatomen substituiertes Phenyl, Phenoxy, Benzyl und Benzyloxy; und
R weiterhin für jeweils gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen steht, für Cycloalkylmethyl oder -ethyl mit 5 bis 7 Kohlenstoffatomen im Cycloalkylteil und Cyclohexylethenyl, schließlich für Alkenyl mit 2 bis 6 Kohlenstoffatomen, für 2-Furyl, für 1,2,4-Triazol-1-yl-methyl, 1,2,4-Triazol-4-yl-methyl, Imidazol-1-yl-methyl und Pyrazol-1-yl-methyl steht,
R' für Wasserstoff, für gegebenenfalls durch Phenyl substituiertes Alkyl mit 1 bis 4 Kohlenstoffatomen, wobei der Phenylrest durch die bei R genannten Phenylsubstituenten substituiert sein kann, sowie für Alkenyl mit 2 bis 4 Kohlenstoffatomen steht,
und deren Säureadditions-Salze und Metallsalz-Komplexe gefunden.

Die Verbindungen der Formel (1) besitzen gegebenenfalls ein asymmetrisches Kohlenstoffatom und können deshalb in den beiden optischen Isomerenformen anfallen.

Weiterhin wurde gefunden, daß man die heterocyclisch substituierten Hydroxyalkyl-azolyl-Derivate der Formel (I) sowie deren Säureadditions-Salze und Metallsalz-Komplexe erhält, wenn man
a) Oxirane der Formel (11) 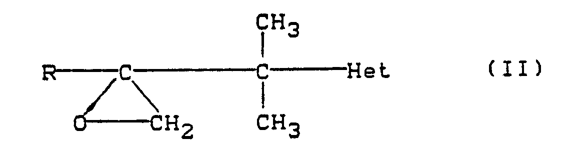 in welcher
   Het und R die oben angegebene Bedeutung haben,

   mit Azolen der Formel (111) 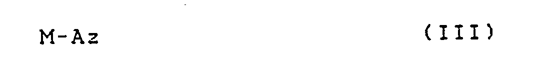 Az die oben angegebene Bedeutung hat und
   M für Wasserstoff oder ein Alkalimetallsalz steht,
   in Gegenwart eines Verdünnungsmittels und gegebenenfalls in Gegenwart einer Base umsetzt, oder
b) Azoloketone der Formel (IV) 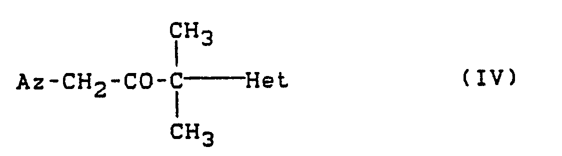 in welcher
   Az und Het die oben angegebene Bedeutung haben,

   mit einer magnesium-organischen Verbindung der Formel (V) 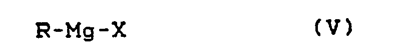 R die oben angegebene Bedeutung hat und
   X für Chlor, Brom oder lod steht,
   in Gegenwart eines Verdünnungsmittels umsetzt,

   oder
c) Azolooxirane der Formel (VI) 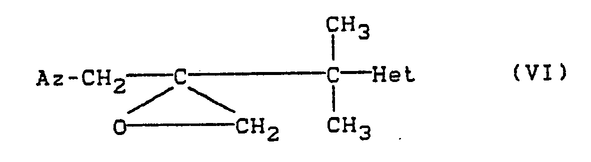 in welcher
   Az und Het die oben angegebene Bedeutung haben, mit gegebenenfalls substituierten Phenolen und Thiophenolen sowie mit metallorganischen Verbindungen der Formel (VII) 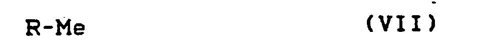 in welcher
   R die oben angegebene Bedeutung hat und
   Me für ein Alkalimetall oder -Mg-X steht, wobei X die oben angegebene Bedeutung hat,

   in Gegenwart eines Verdünnungsmittels und gegebenenfalls in Gegenwart einer Base umsetzt, und gegebenenfalls noch
d) die nach den Verfahren a), b) oder c) erhaltenen Hydroxy-Verbindungen in Gegenwart eines Verdünnungsmittels in das Alkalimetall-alkoholat überführt und dieses mit einem Halogenid zu den entsprechenden Ether-Derivaten umsetzt,

und gegebenenfalls noch an die so erhaltenen Verbindungen der Formel (I) anschließend eine Säure oder ein Metallsalz addiert.

Die neuen, heterocyclisch substituierten Hydroxyalkylazolyl-Derivate der Formel (I) weisen starke fungizide Eigenschaften auf. Dabei zeigen überraschenderweise die erfindungsgemäßen Verbindungen eine höhere Wirkung als die aus dem Stand der Technik bekannten Hydroxyethyl-azolyl-Derivate 3,3-Dimethyl-1-phenoxy-2-(1,2,4-triazol-1-yl-methyl)-2-butanol bzw. 1-(2-Chlor-4-fluorphenoxy)-3,3-dimethyl-2-(1,2,4-triazol-1-yl-methyl)-2-butanol oder 1-(4-Chlorphenoxy)-2-(2,4-dichlorphenyl)-3-(imidazol-1-yl)-2-propanol, welche konstitutionell und wirkungsmäßig naheliegende Verbindungen sind. Die erfindungsgemäßen Stoffe stellen somit eine Bereicherung der Technik dar.

Die erfindungsgemäßen heterocyclisch substituierten Hydroxyalkyl-azolyl-Derivate sind durch die Formel (I) allgemein definiert.

Bevorzugt sind diejenigen Verbindungen der Formel (1), in denen
Az für 1,2,4-Triazol-1 -yl, 1,2,4-Triazol-4-yl oder Imidazol-1-yl steht;
Het für jeweils gegebenenfalls einfach bis dreifach, gleich oder verschieden substituiertes Dioxolan-2-yl, 1,3-Dioxan-5-yl oder 1,3-Dioxan-2-yl steht, wobei als Substituenten beispielsweise genannt seien: Methyl, Ethyl, n-Propyl, Isopropyl sowie jeweils gegebenenfalls einfach bis dreifach, gleich oder verschieden durch Fluor, Chlor, Methyl, Trifluormethyl oder Trifluormethoxy substituiertes Phenyl und Phenoxymethyl;
R für tert.-Butyl, Trimethyl-propyl, Tetramethyl-propyl sowie für jeweils gegebenenfalls einfach bis dreifach, gleich oder verschieden substituiertes Phenyl, Benzyl, Phenethyl, Phenoxymethyl, Phenylthiomethyl oder Phenethenyl steht, wobei als Phenylsubstituenten beispielsweise genannt seien: Fluor, Chlor, Methyl, Trifluormethyl, Trifluormethoxy, Trifluormethylthio, Hydroximinomethyl, 1-Hydroximinoethyl, Methoximinomethyl und 1-Methoximinoethyl sowie jeweils gegebenenfalls durch Chlor und/oder Methyl substituiertes Phenyl, Phenoxy, Benzyl und Benzyloxy; R ferner für jeweils gegebenenfalls einfach bis zweifach, gleich oder verschieden durch Methyl, Ethyl oder Isopropyl substituiertes Cyclohexyl, Cyclohexylmethyl, Cyclohexylethyl oder Cyclohexylethenyl steht; R weiterhin für Allyl, Dimethyloropenyl, 2-Furyl, 1,2,4-Triazol-1-yl-methyl, 1,2,4-Triazol-4-yl-methyl, Imidazol-1-yl-methyl oder Pyrazol-1-yl-methyl steht; und
R' für Wasserstoff, Methyl, 4-Chlorbenzyl oder Allyl steht.

Bevorzugte erfindungsgemäße Verbindungen sind auch Additionsprodukte aus Säuren und denjenigen heterocyclisch substituierten Hydroxyalkyl-azolyl-Derivaten der Formel (I), in denen die Substituenten Az, Het, R und R' die Bedeutungen haben, die bereits für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalin-disulfonsäure.

Außerdem bevorzugte erfindungsgemäße Verbindungen sind Additionsprodukte als Salzen von Metallen der II. bis IV. Haupt- und der und II. sowie IV. bis VIII. Nebengruppe und denjenigen heterocyclisch substituierten Hydroxyalkylazolyl-Derivaten der Formel (I), in denen die Substituenten Het, R und R' die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Verbindungen der Formel (la) genannt:

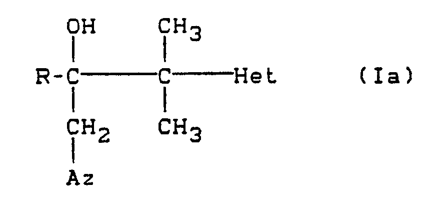

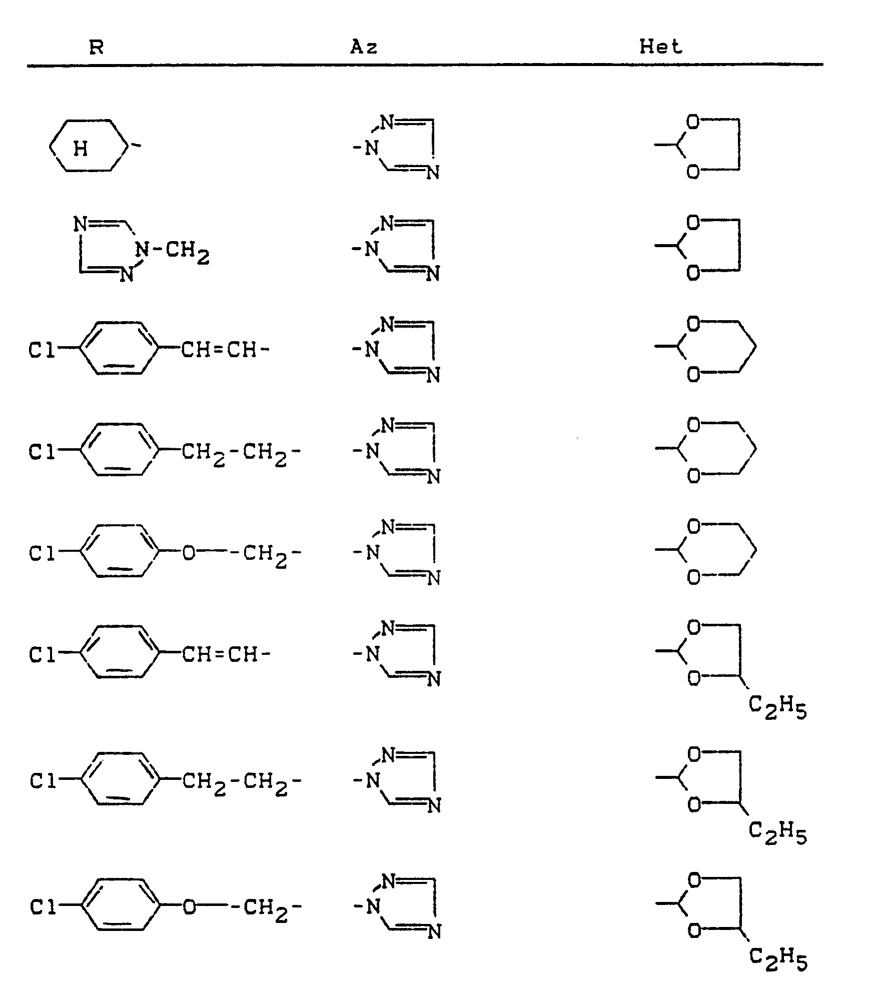

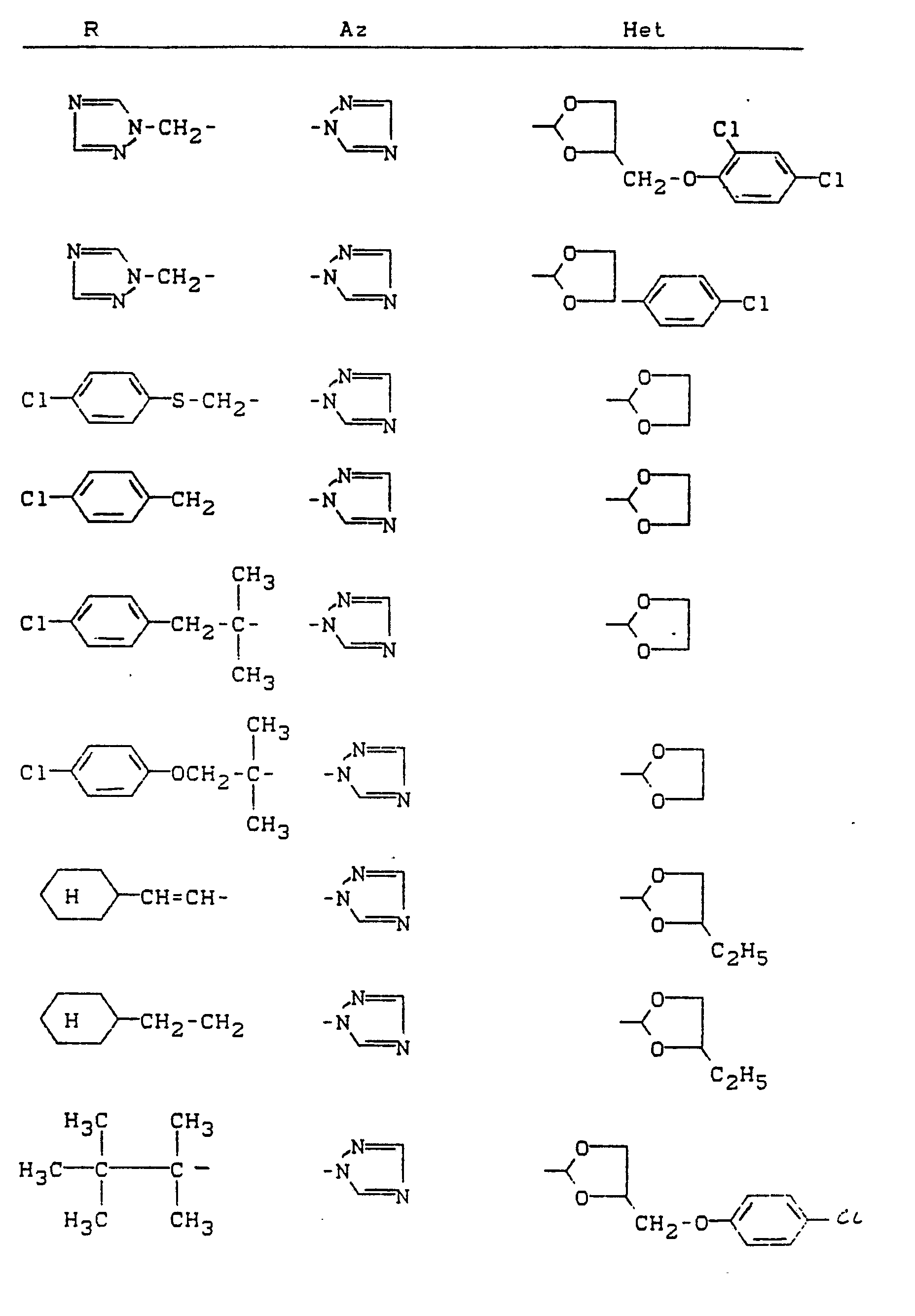

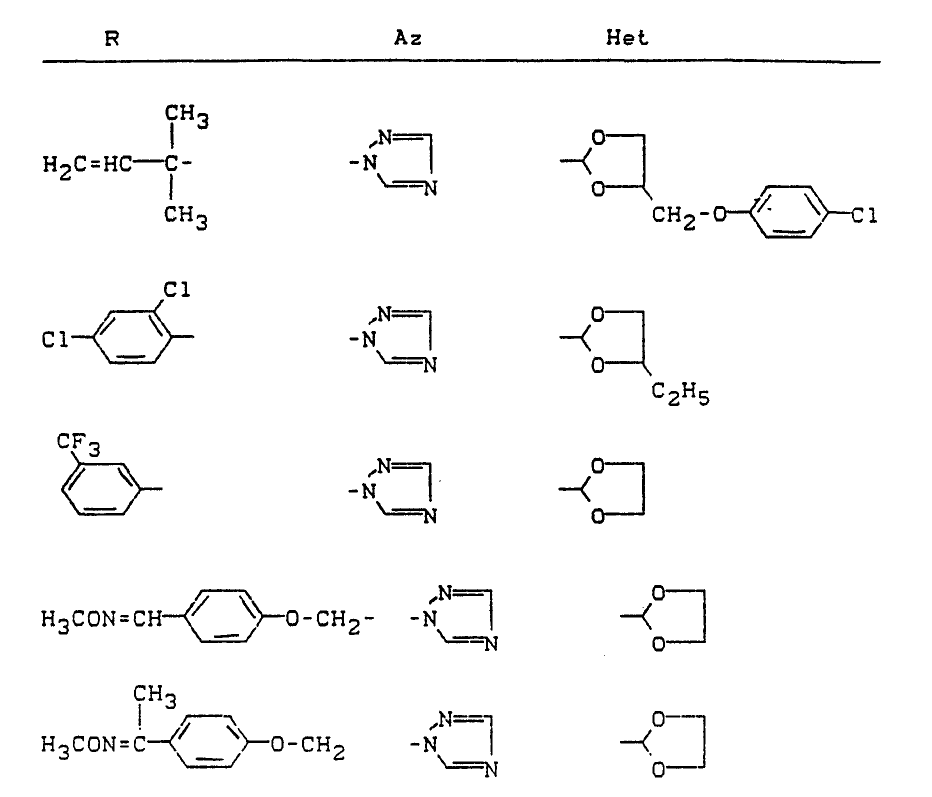

Verwendet man beispielsweise 2-[2-(1,3-Dioxolan-2-yl)-prop-2-yl]-2-(4-fluorphenoxymethyl)-oxiran und 1,2,4-Triazol als Ausgangsstoffe, so kann der Ablauf des erfindungsgemäßen Verfahrens (a) durch das folgende Formelschema wiedergegeben werden:

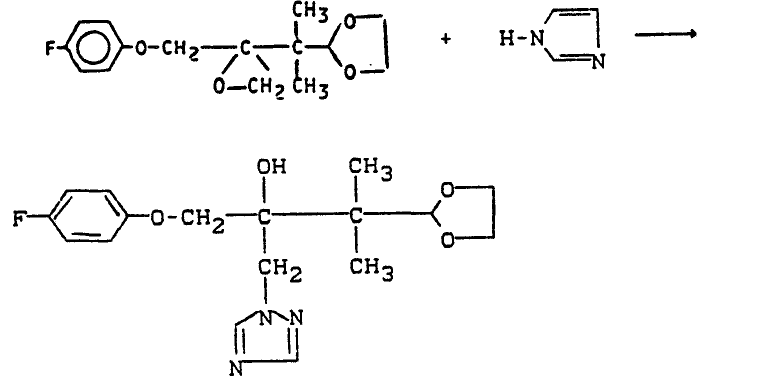

Verwendet man beispielsweise 2-(1,3-Dioxolan-2-yl)-prop-2-yl-(1,2,4-triazol-1-yl-methyl)-keton und 4-Chlorbenzylmagnesiumchlorid als Ausgangsstoffe, so kann der Ablauf des erfindungsgemäßen Verfahrens (b) durch das folgende Formelschema wiedergegeben werden:

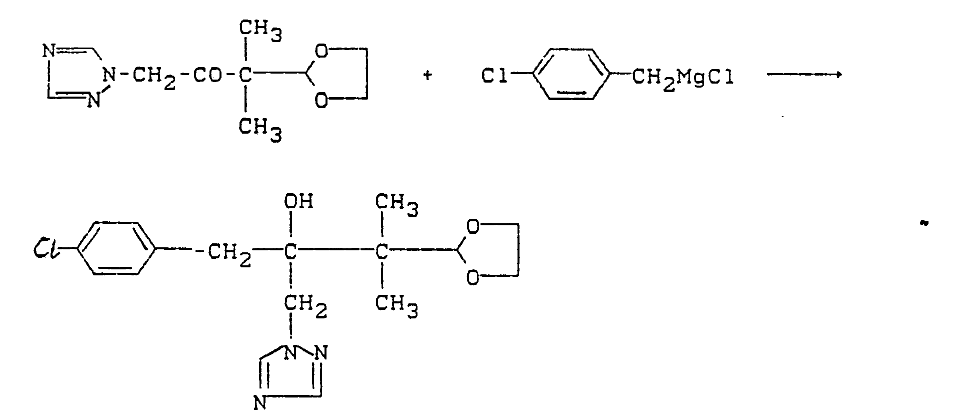

Verwendet man beispielsweise 2-[2-(1,3-Dioxolan-2-yl)-prop-2-yl]-2-(1,2,4-triazol-1-yl-methyl)-oxiran und 4-Fluorphenol als Ausgangsstoffe, so kann der Ablauf des erfindungsgemäßen Verfahrens (c) durch das folgende Formelschema wiedergegeben werden:

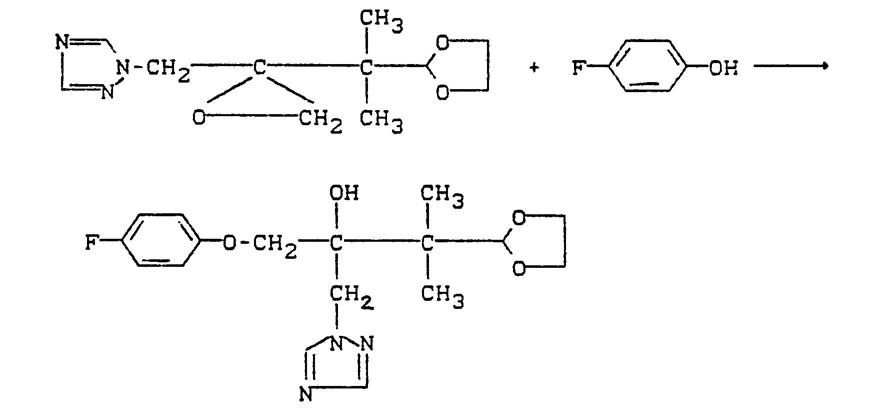

Die für die Durchführung des erfindungsgemäßen Verfahrens (a) als Ausgangsstoffe zu verwendenden Oxirane sind durch die Formel (II) allgemein definiert. In dieser Formel haben Het und R die Bedeutungen, die bereits im Zusammenhang mit der Beschreibung der erfindungsgemäßen Stoffe der Formel (I) für diese Substituenten genannt wurden.

Die Oxirane der Formel (II) sind noch nicht bekannt. Sie können jedoch in allgemein bekannter Art und Weise erhalten werden, indem man Ketone der Formel (VIII) 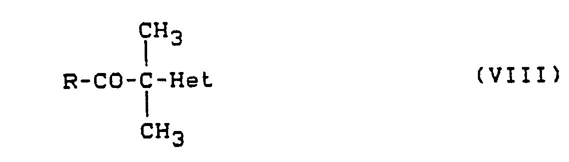 in welcher
Het und R die oben angegebene Bedeutung haben,

entweder
a) mit Dimethyloxosulfonium-methylid der Formel (IX) r 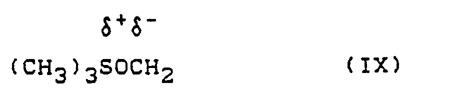 in Gegenwart eines Verdünnungsmittels umsetzt, oder
β) mit Trimethylsulfonium-methylsulfat der Formel (X) 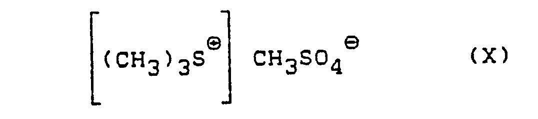 in Gegenwart eines inerten organischen Lösungsmittels sowie in Gegenwart einer Base umsetzt.

Die bei der Herstellung der Oxirane der Formel (11) als Ausgangsstoffe benötigten Ketone der Formel (VIII) sind teilweise bekannt (vgl. z.B. J.Org.Chem. 32, 404 (1967)) bzw. sind sie Gegenstand eigener älterer Patentanmeldungen (vgl. EP-A 0 097 882 und EP-A 0 097 881) bzw. können sie in bekannter Art und Weise erhalten werden, indem man 1-(N-Morpholino)-isobuten der Formel (XI) 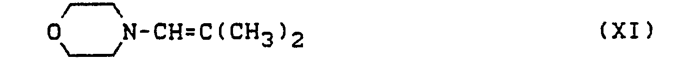 mit Chloriden der Formel (XII) 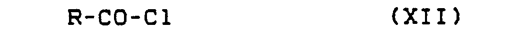 in welcher
R die oben angegebene Bedeutung hat,

in Gegenwart eines Lösungsmittels, wie beispielsweise Diethylether, bei Temperaturen zwischen 20° C und 120°C umsetzt und die so erhaltenen Keto-Derivate der Formel (XIII) 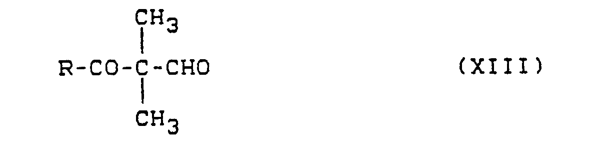 in welcher
R die oben angegebene Bedeutung hat, in üblicher Weise an der Aldehydgruppe mit entsprechenden Diolen in Gegenwart eines inerten organischen Lösungsmittels, wie beispielsweise Toluol, und in Gegenwart einer starken Säure als Katalysator, wie beispielsweise p-Toluolsulfonsäure, bei Temperaturen zwischen 80° C und 110° C derivatisiert.

Ketone der Formel (VIII) mit R - gegebenenfalis substituiertes Phenethenyl oder Cyclohexylethenyl können auch erhalten werden, indem man entsprechende Benzaldehyde oder Cyclohexylcarbaldehyde mit entsprechenden Methylketonen in üblicher Weise einer Aldolkondensation unterwirft. Die dabei entstehenden Ketone der Formel (VIII) mit R = gegebenenfalls substituiertes Phenethenyl oder Cyclohexylethenyl können gegebenenfalls in üblicher Weise zu Ketonen der Formel (VIII) mit R = gegebenenfalls substituiertes Phenethyl oder Cyclohexylethyl hydriert werden (vgl. auch die Herstellungsbeispiele).

Das bei der Verfahrensvariante (a) benötigte Dimethyloxosulfoniummethylid der Formel (IX) ist bekannt (vgl. J. Amer. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid bzw. Natriumamid oder Kalium-tert.-Butylat in Gegenwart eines Verdünnungsmittels erzeugt.

Das bei der Verfahrensvariante (ß) benötigte Trimethylsulfoniummethylsulfat der Formel (X) ist ebenfalls bekannt (vgl. Heterocycles 8, 397 (1977)). Es wird bei der obigen Umsetzung ebenfalls in frisch hergestelltem Zustand eingesetzt, indem man es durch Reaktion von Dimethylsulfid mit Dimethylsulfat in situ erzeugt.

Bei der Variante (a) des Verfahrens zur Herstellung der Oxirane der Formel (11) kommt als Verdünnungsmittel vorzugsweise Dimethylsulfoxid in Frage.

Die Reaktionstemperaturen können bei der oben beschriebenen Verfahrensvariante (a) innerhalb eines größeren Bereiches variiert werden. Im allgemeinen arbeitet man bei Temperaturen zwischen 20° C und 80° C.

Die Durchführung des Verfahrens zur Herstellung der Oxirane der Formel (II) nach der Variante (a) und die Aufarbeitung des bei dieser Synthese anfallenden Reaktionsgemisches erfolgen nach üblichen Methoden (vgl. J. Amer. Chem. Soc. 87,1363-1364 (1965)).

Bei der Variante (ß) zur Herstellung der Oxirane der Formel (II) kommt als inertes organisches Lösungsmittel vorzugsweise Acetonitril in Betracht.

Als Basen können bei der Verfahrensvariante (ß) starke anorganische oder organische Basen verwendet werden. Vorzugsweise in Frage kommt Natriummethylat.

Die Reaktionstemperaturen können bei der oben beschriebenen Verfahrensvariante (ß) innerhalb eines bestimmten Bereiches variiert werden. Im allgemeinen arbeitet man bei Temperaturen zwischen 0°C und 60°C, vorzugsweise bei Raumtemperatur.

Die Durchführung des Verfahrens zur Herstellung der Oxirane der Formel (11) nach der Variante (ß) und die Aufarbeitung des bei dieser Synthese anfallenden Reaktionsproduktes erfolgen nach üblichen Methoden (vgl. Heterocycles 8, 397 (1977)).

Die Oxirane der Formel (11) können bei dem erfindungsgemäßen Verfahren gegebenenfalls ohne Isolierung direkt weiter umgesetzt werden.

Die außerdem für das erfindungsgemäße Verfahren (a) als Ausgangsstoffe zu verwendenden Azole sind durch die Formel (111) allgemein definiert. In dieser Formel steht Az für die Bedeutungen, die bereits in der Erfindungsdefinition für diesen Substituenten genannt wurden. M steht vorzugsweise für Wasserstoff, Natrium oder Kalium.

Die Azole der Formel (111) sind allgemein bekannte Verbindungen der organischen Chemie.

Die bei der Durchführung des erfindungsgemäßen Verfahrens ((b) als Ausgangsstoffe zu verwendenden Azolketone sind durch die Formel (IV) allgemein definiert. In dieser Formel haben Az und Het die Bedeutungen, die bereits im Zusammenhang mit der Beschreibung der erfindungsgemäßen Stoffe der Formel (I) für diese Substituenten genannt wurden.

Die Azoloketone der Formel (IV) sind teilweise bekannt (vgl. beispielsweise EP-A 00 43 923), teilweise sind sie jedoch Gegenstand einer eigenen älteren Patentanmeldung (vgl. EP-A 0 097 881). Die Azoloketone der Formel (IV) können in allgemein bekannter Art und Weise erhalten werden, indem man Halogenmethyl-Ketone der Formel (XV) 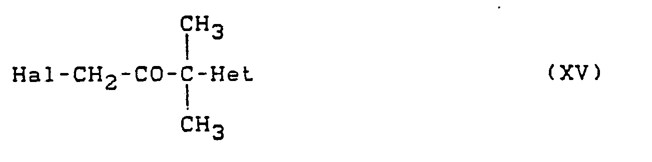 in welcher
Het die oben angegebene Bedeutung hat und
Hal für Chlor oder Brom steht,

in üblicher Weise mit 1,2,4-Triazol oder Imidazol in Gegenwart eines inerten organischen Lösungsmittels, wie beispielsweise Aceton, und in Gegenwart eines Säurebindemittels, wie beispielsweise Kaliumcarbonat, bei Temperaturen zwischen 20°C und 150°C umsetzt.

Die bei der Herstellung der Azoloketone der Formel (IV) als Ausgangsstoffe benötigten Halogenmethyl-Ketone der Formel (XV) sind noch nicht bekannt; auch sie sind Gegenstand eigenen älteren Patentanmeldung (vgl. EP-A 0 097 881). Sie können gemäß der oben beschriebenen Herstellung der Ketone der Formel (VIII) erhalten werden.

Die außerdem für das erfindungsgemäße Verfahren (b) als Ausgangsstoffe zu verwendenden magnesium-organischen Verbindungen sind durch die Formel (V) allgemein definiert. In dieser Formel steht R für die Bedeutungen, die bereits im Zusammenhang mit der Beschreibung der erfindungsgemäßen Stoffe der Formel (I) für diesen Substituenten genannt wurden.

Die magnesium-organischen Verbindungen der Formel (V) sind allgemein bekannte Verbindungen der organischen Chemie (sogenannte "Grignard-Verbindungen"); bzw. werden sie in allgemein bekannter Art und Weise erhalten.

Die bei der Durchführung des erfindungsgemäßen Verfahrens (c) als Ausgangsstoffe zu verwendenden Azolooxirane sind durch die Formel (VI) allgemein definiert. In dieser Formel haben Az und Het die Bedeutungen, die bereits im Zusammenhang mit der Beschreibung der erfindungsgemäßen Stoffe der Formel (I) für diese Substituenten genannt wurden.

Die Azolooxirane der Formel (VI) sind noch nicht bekannt; sie können jedoch in allgemein bekannter Art und Weise erhalten werden, indem man Azoloketone der Formel (IV) entsprechend den oben angegebenen Verfahrensvarianten (a) und (ß) epoxidiert.

Die außerdem für das erfindungsgemäße Verfahren (c) als Ausgangsstoffe in Frage kommenden Phenole und Thiophenole ergeben sich aus der Definition des Restes R bei den erfindungsgemäßen Stoffen der Formel (I).

Die weiterhin für das erfindungsgemäße Verfahren (c) als Ausgangsstoffe zu verwendenden metallorganischen Verbindungen sind durch die Formel (VII) allgemein definiert. In dieser Formel steht R für die Bedeutungen, die bereits im Zusammenhang mit der Beschreibung der erfindungsgemäßen Stoffe der Formel (I) für diesen Substituenten genannt wurden. Me steht vorzugsweise für Lithium, Natrium, Kalium und die Gruppierung -Mg-X, wobei X für Chlor, Brom oder lod steht.

Die Phenole und Thiophenole sowie die metallorganischen Verbindungen der Formel (VII) sind allgemein bekannte Verbindungen der organischen Chemie.

Als Verdünnungsmittel kommen für das erfindungsgemäße Verfahren (a) unter den Reaktionsbedingungen inerte organische Lösungsmittel in Frage. Hierzu gehören vorzugsweise Alkohole, wie z.B. Ethanol, Methoxyethanol oder Propanol Ketone, wie z.B. 2-Butanon; Nitrile, wie z.B. Acetonitril Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für das erfindungsgemäße Verfahren (a) alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens (a) in einem größeren Bereich variiert werden. Im allgemeinen arbeitet man bei Temperaturen zwischen 0° C und 200° C, vorzugsweise zwischen 60° C und 150°C.

Bei der Durchführung des erfindungsgemäßen Verfahrens (a) setzt man vorzugsweise auf 1 Mol Oxiran der Formel (11) 1 bis 2 Mol Azol und gegebenenfalls 1 bis 2 Mol Base ein. Die Isolierung der Endprodukte erfolgt in allgemein üblicher Weise.

Als Verdünnungsmittel kommen für das erfindungsgemäße Verfahren (b) alle für eine Grignard-Reaktion üblichen Lösungsmittel in Frage. Hierzu gehören vorzugsweise Ether, wie Diethylether oder Tetrahydrofuran, sowie Gemische mit anderen organischen Solventien, wie beispielsweise Benzol.

Die Reaktionstemperaturen können bei der Durchführung des erfindungsgemäßen Verfahrens (b) in einem größeren Bereich variiert werden. Im allgemeinen arbeitet man zwischen etwa 20°C bis 120°C, vorzugsweise zwischen etwa 30° C bis etwa 80° C.

Bei der Durchführung des Verfahrens (b) setzt man auf 1 Mol Azoloketon der Formel (IV) vorzugsweise einen Überschuß von 3 bis 5 Mol einer metallorganischen Verbindung der Formel (V) ein. Die Isolierung der Endprodukte erfolgt in allgemein üblicher Weise.

Als Verdünnungsmittel kommen für das erfindungsgemäße Verfahren (c) unter den Reaktionsbedingungen inerte organische Lösungsmittel in Frage. Hierzu gehören vorzugsweise Alkohole, wie z.B. Ethanol, Methoxyethanol oder Propanol Ketone, wie z.B. 2-Butanon; Nitrile, wie z.B. Acetonitril: Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für das erfindungsgemäße Verfahren (c) alle üblicherweise verwendbaren anorganischen und organischen Basen in Betracht. Hierzu gehören vorzugsweise die beim Verfahren (a) bereits genannten Verbindungen.

Die Reaktionstemperaturen können bei der Durchführung des erfindungsgemäßen Verfahrens (c) in einem größeren Bereich variiert werden. Im allgemeinen arbeitet man bei Temperaturen zwischen 0°C und 200°C, vorzugsweise zwischen 60°C und 150° C.

Bei der Durchführung des erfindungsgemäßen Verfahrens (c) setzt man vorzugsweise auf 1 Mol Azolooxiran der Formel (VI) 1 bis 2 Mol (Thio)Phenol und gegebenenfalls 1 bis 2 Mol Base bzw. 1 bis 3 Mol der metallorganischen Verbindungen der Formel (VII) ein. Die Isolierung der Endprodukte erfolgt jeweils in allgemein üblicher Weise.

Zur Herstellung der Ether-Verbindungen der Formel (I) nach dem erfindungsgemäßen Verfahren (d) wird zweckmäßigerweise so vorgegangen, daß man Verbindungen der Formel (I) in einem geeigneten inerten organischen Lösungsmittel mittels Alkalimetall-hydrid oder -amid in das Alkalimetall-alkoholat überführt und dieses ohne Isolierung sofort mit einem entsprechenden Halogenid, wie insbesondere einen Alkylhalogenid im Temperaturbereich zwischen 0 und 80°C umsetzt, wobei unter Austritt von Alkalihalogenid die Ether der Verbindungen der Formel (I) in einem Arbeitsgang erhalten werden.

In einer bevorzugten Ausführungsform werden zweckmäßigerweise die Herstellung der Alkoholate sowie deren weitere Umsetzung mit einem Halogenid in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise Ammonium- oder Phosphonium-Verbindungen, durchgeführt, wobei in der organischen Phase oder an der Grenzfläche die Alkoholate entstehen und mit den in der organischen Phase befindlichen Halogeniden umgesetzt werden.

Die nach den erfindungsgemäßen Verfahren a), b) und c) erhältlichen Hydroxy-Verbindungen (wobei in Formel (I) R' für Wasserstoff steht) können auch in die entsprechenden Ester überführt werden.

Die Verbindungen stellen somit interessante Zwischenprodukte dar.

Zur Herstellung der Ester wird zweckmäßigerweise so verfahren, daß man die Hydroxy-Verbindungen z.B. mit Säurehalogeniden in Gegenwart eines inerten organischen Lösungsmittels, wie beispielsweise Essigester, bei Temperaturen zwischen 0°C und 100°C umsetzt; oder mit Säureanhydriden in Gegenwart eines inerten organischen Lösungsmittels, wie beispielsweise Methylenchlorid oder Überschuß an Säureanhydrid, und in Gegenwart eines Katalysators, wie beispielsweise Natriumacetat, bei Temperaturen zwischen 0°C und 150°C umsetzt.

Die Verbindungen der Formel (1) können auch in Säureadditions-Salze bzw. Metallsalz-Komplexe überführt werden.

Die Säureadditions-Salze der Verbindungen der Formel (I) können in einfacher Weise nach üblichen Salzbildungsmethoden, z.B. durch Lösung einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metall-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Puccinia-Arten, wie gegen den Erreger des Braunrostes am Weizen (Puccinia recondita), und von Botrytis-Arten, wie gegen den Grauschimmel (Botrytis cinerea) sowie auch zur Bekämpfung von Mehltau, Leptosphaeria nodorum, Cochliobolus sativus und Pyrenophora teres an Getreide und gegen Pyricularia und Pellicularia an Reis eingesetzt werden.

In entsprechenden Aufwandmengen zeigen die erfindungsgemäßen - Stoffe auch eine wachstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen wie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkoholether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Metylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe. wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0.001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

### Herstellungsbeispiele:

### Beispiel 1

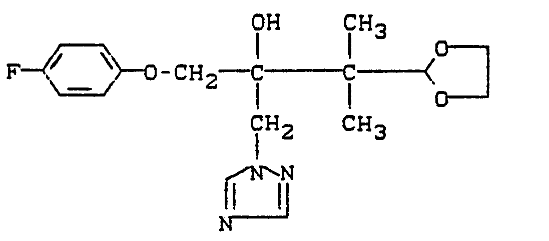

### (Verfahren a)

Eine Lösung von 17,6 g (0,0623 Mol) 2-[2-(1,3-Dioxolan-2-yl)-prop-2-yl]-2-(4-fluorphenoxymethyl)-oxiran in 30 ml n-Propanol werden bei Raumtemperatur zu einer Lösung von 4,93 g (0,0715 Mol) 1,2,4-Triazol und 0,36 g (0,0065 Mol) Kaliumhydroxid in 30 ml n-Propanol getropft. Das Reaktionsgemisch wird 2 Tage unter Rückfluß erhitzt und anschließend eingeengt. Der Rückstand wird in Essigester aufgenommen, zweimal mit Wasser und einmal mit gesättigter Kochsalzlösung gewaschen, über Natriumsulfat getrocknet und eingeengt. Der Rückstand wird säulenchromatographisch gereinigt (Dichlormethan/Essigester = 3:1) und aus wenig Ether umkristallisiert.

Man erhält 10,2 g (47 % der Theorie) 3-(1,3-Dioxolan-2-yl)-1-(4-fluorphenoxy)-3-methyl-2-(1,2,4-triazol-1-yl- methyl)-2-butanol vom Schmelzpunkt 102°C bis 104°C.

### Herstellung des Ausgangsproduktes:

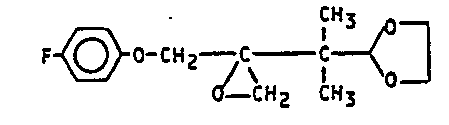

Eine Suspension von 16,47 g (0,0748 Mol) Trimethylsulfoxoniumiodid in 20 ml absolutem Dimethylsulfoxid wird bei Raumtemperatur mit 8,4 g (0,0748 Mol) Kalium-tert.butylat versetzt. Man läßt 30 Minuten nachrühren und tropft dann eine Lösung von 3-(1,3-Dioxolan-2-yl)-1-(4-fluorphenoxy)-3-methyl-2-butanon in 20 ml absolutem Toluol zu. Das Reaktionsgemisch wird über Nacht bei Raumtemperatur gerührt, danach 2 Stunden auf 50°C erhitzt, abgekühlt und mit Wasser und Toluol versetzt. Die Toluolphase wird abgetrennt, zweimal mit Wasser und einmal mit gesättigter Natriumchloridlösung gewaschen, über Natriumsulfat getrocknet und eingeengt.

Man erhält 17,6 g (91 % der Theorie) 2-[2-(1,3-Dioxolan-2-yl)-prop-2-yl]-2-(4-fluorphenoxymethyl)-oxiran als Öl, das direkt weiter umgesetzt wird.

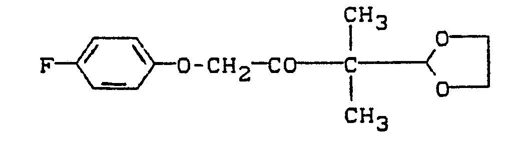

Ein Gemisch aus 16,7 g (0,15 Mol) 4-Fluorphenol, 29 g (0,15 Mol) 1-Chlor-3-(1,3-dioxolan-2-yl)-3-methyl-2-butanon und 23,4 g (0,17 Mol) gepulvertem Kaliumcarbonat in 300 ml Methylethylketon wird 16 Stunden unter Rückfluß erhitzt. Man läßt abkühlen und filtriert. Das Filtrat wird eingeengt, der Rückstand mit Dichlormethan aufgenommen, einmal mit 5 %iger Natronlauge und einmal mit Wasser gewaschen, über Natriumsulfat getrocknet, eingeengt und destilliert. Man erhält 29 g (72 % der Theorie) 3-(1,3-Dioxolan-2-yl)-1-(4- fluorphenoxy)-3-methyl-2-butanon vom Siedepunkt 143° bis 145° C/0,1 mbar.

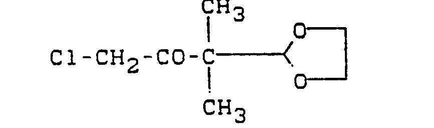

204 g (1,38 Mol) 4-Chlor-2,2-dimethyl-3-keto-butanal werden mit 93 g (1,5 Mol) Ethylenglykol und 0,7 g p-Toluolsulfonsäure in 400 ml Methylenchlorid während 3 Stunden am Wasserabscheider erhitzt. Die organische Phase wird mit 150 ml 5 %iger Natronlauge und danach mit 400 ml Wasser extrahiert. Das Lösungsmitel wird abdestilliert und der Rückstand im Wasserstrahlvakuum destilliert.

Man erhält 211 g (79,8 % der Theorie) 1-Chlor-3-(dioxolan-2-yl)-3-methyl-2-butanon vom Siedepunkt 127°C bis 128°C/14 mbar.

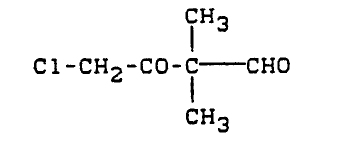

210 g (1,5 Mol) 1-(N-Morpholino)-isobuten werden innerhalb einer Stunde zu 169 g (1,5 Mol) Chloracetylchlorid, gelöst in 350 ml Diethylether, bei 5°C zugetropft. Nach beendeter Zugabe rührt man weitere 3 Stunden unter Rückflußkühlung. Die Lösung wird auf 100 g Eis gegossen, mit wäßriger Natriumhydrogencarbonatlösung auf pH 5 gebracht und die Etherphase abgetrennt. Die Wasserphase wird mit 100 ml Diethylether extrahiert, die organischen Phasen werden vereinigt, über Natriumsulfat getrocknet, das Lösungsmittel abdestilliert und der Rückstand im Wasserstrahlvakuum destilliert.

Man erhält 136,4 g (61 % der Theorie) 4-Chlor-2,2-dimethyl-3-ketobutanal vom Siedepunkt 95°C bis 98°C/14 mbar.

### Beispiel 2 und 3

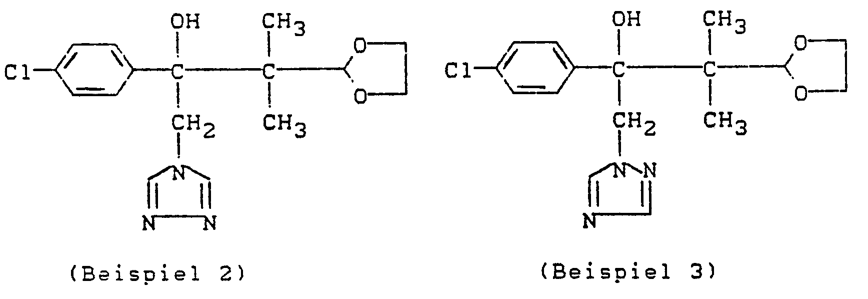

### ^{»} (Verfahren a)

Eine Lösung von 26,8 g (0,1 Mol) 2-(4-Chlorphenyl)-2-[2-(1,3-dioxolan-2-yl)-prop-2-yl]-oxiran, 7,6 g (0,11 Mol) 1,2,4-Triazol und 0,5 g Kaliumhydroxid in 200 ml absolutem Butanol wird 18 Stunden unter Rückfluß erhitzt. Man läßt auf Raumtemperatur abkühlen und versetzt mit 800 ml Wasser. Die organische Phase wird abgetrennt, über Natriumsulfat getrocknet und eingeengt. Der Rückstand wird mit 200 ml Isopropylether/Essigester (10:1) verrührt.

Der ausfallende Feststoff wird abfiltriert und getrocknet. Man erhält 4,5 g (13,3 % der Theorie) 3-(1,3-Dioxolan-2-yl)-2-(4-chlorphenyl)-3-methyl-1-(1,2,4-triazol-4-yl)-2-butanol (Beispiel 2) vom Schmelzpunkt 180°C bis 82° C.

Das Filtrat wird eingeengt und der Rückstand zunächst mit 350 ml Aceton aufgenommen und dann mit 8 g 1,5-Naphthalin-disulfonsäure in 30 ml Aceton versetzt. Man läßt 6 Stunden bei 0°C rühren, saugt den Feststoff ab und versetzt mit gesättigter, wäßriger Natriumhydrogencarbonat/Methylenchlorid-Lösung. Die organische Phase wird abgetrennt, über Natriumsulfat getrocknet und eingeengt.

Man erhält 19 g (56,2 % der Theorie) 3-(1,3-Dioxolan-2-yl)-2-(4-chlorphenyl)-3-methyl-1-(1,2,4-triazol-1-yl)-2-butanol (Beispiel 3) als Öl.

### Herstellung des Ausgangsproduktes:

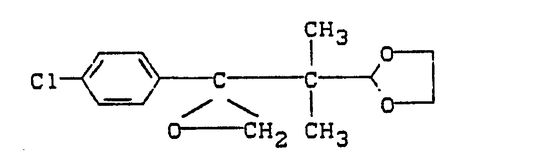

29,5 g (0,208 Mol) Methyliodid werden zu 13,7 g (0,22 Mol) Dimethylsulfid in 130 ml absolutem Dimethylsulfoxid und 120 ml absolutem Tetrahydrofuran getropft, wobei die Temperatur auf ca. 30°C steigt. Man läßt 5 Stunden nachrühren und versetzt dann mit einer Lösung von 33 g (0,13 Mol) 4-Chlorphenyl-2-[2-(1,3-dioxolan-2-yl)-prop-2-yl]-keton in 100 ml absolutem Toluol. Innerhalb einer Stunde werden 9,5 g Natriummethylat portionsweise zugegeben. Man läßt das Reaktionsgemisch 3 Stunden nachrühren und gibt innerhalb von 30 Minuten nochmals 5,6 g Natriummethylat in 2 Portionen zu. Man läßt über Nacht rühren und gibt das Reaktionsgemisch auf 700 ml Eiswasser. Die organische Phase wird abgetrennt und die wäßrige Phase mit 200 ml Toluol ausgeschüttelt. Die vereinigten organischen Phasen werden zweimal mit je 1000 ml Wasser gewaschen, über Natriumsulfat getrocknet und eingeengt. Der ölige Rückstand wird im Vakuum entgast.

Man erhält 26,8 g 2-(4-Chlorphenyl)-2-[2-(1,3-dioxolan-2-yl)-prop-2-yl]-oxiran als Öl, das direkt weiter umgesetzt wird.

### Beispiel 4

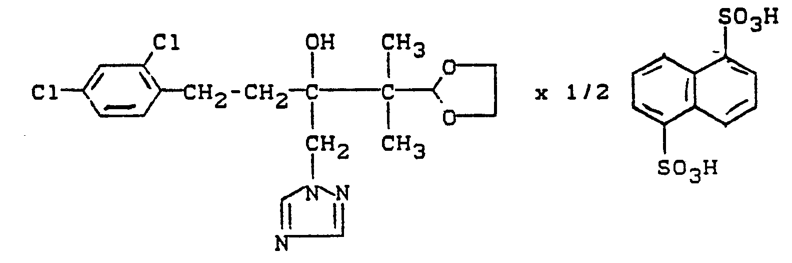

### (Verfahren a / Salzbildung)

13,8 g (0,2 Mol) 1,2,4-Triazol werden portionsweise zu einer Suspension von 6 g (80 %ig, 0,2 Mol) Natriumhydrid in 330 ml absolutem Dimethylformamid gegeben. Man läßt 30 Minuten nachrühren und versetzt dann mit 45 g (0,136 Mol) 2-(2,4-Dichlorphenylethyl)-2-[2-(1,3-dioxolan-2-yl)-prop-2-yl]-oxiran in 80 ml Dimethylformamid. Das Reaktionsgemmisch wird 4 Stunden bei 80° C gerührt. Danach läßt man abkühlen und gibt auf 600 ml Eiswasser/800 ml Methylenchlorid. Die Methylenchloridphase wird abgetrennt, zweimal mit je 1500 ml Wasser gewaschen, über Natriumsulfat getrocknet und eingeengt. Der ölige Rückstand wird in 400 ml Aceton aufgenommen und bei 0°C mit 14,4 g 1,5-Naphthalin-disulfonsäure in 40 ml Aceton versetzt. Man läßt 5 Stunden nachrühren und saugt den entstandenen Niederschlag ab.

Man erhält 43,1 g (29,1 % der Theorie) 1-(2,4-Dichlorphenyl)-4-(1,3-dioxolan-2-yl)-4-methyl-3-(1,2,4-triazol-1-yl-methyl)-3-pentanol-naphthalindisulfonat-(1,5) vom Schmelzpunkt 183°C.

### Herstellung des Ausgangsproduktes:

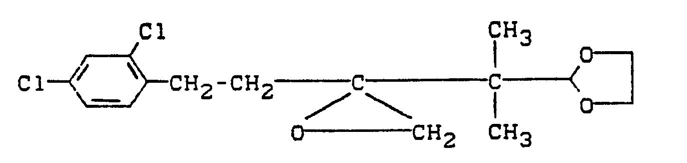

Gemäß Beispiel 3 erhält man durch Umsetzung von 83 g (0,262 Mol) 2,4-Dichlorphenylethyl-2-(1,3-dioxolan-2-yl)-prop-2-yl-keton mit 28,2 g (0,455 Mol) Dimethylsulfid/60 g Methyliodid 90 g 2-(2,4-Dichlorphenylethyl)-2-[2-(1,3-dioxolan-2-yl)-prop-2-yl]-oxiran als Öl, das direkt weiter umgesetzt wird.

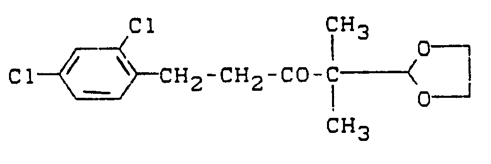

100 g 2,4-Dichlorphenylethenyl-2-(1,3-dioxolan-2-yl)-prop-2-yl-keton werden mit 10 g Raney-Nickel in 600 ml Tetrahydrofuran 25 Minuten bei einem Druck von 55 bar auf 25°C erhitzt. Das Reaktionsgemisch wird anschließend eingeengt und der Rückstand im Vakuum destilliert.

Man erhält 83 g 2,4-Dichlorphenylethyl-2-(1,3-dioxolan-2-yl)-prop-2-yl-keton vom Siedepunkt 148°C/0,1 mbar.

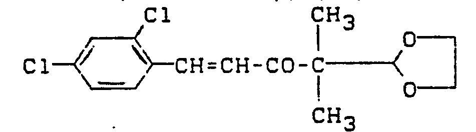

196 g (1,2 Mol) 2,4-Dichlorbenzaldehyd und 178 g (1,125 Mol) Methyl-2-(1,3-dioxolan-2-yl)-prop-2-yl-keton in 400 ml Ethanol und 140 ml Wasser werden tropfenweise mit 95 ml 10 %iger Natronlauge versetzt. Man läßt das Reaktionsgemisch 10 Stunden nachrühren, saugt den entstandenen Feststoff ab und wäscht ihn mit Ethanol.

Man erhält 309 g 2,4-Dichlorphenylethenyl-2-(1,3-dioxolan-2-yl)-prop-2-yl-keton vom Schmelzpunkt 92°C bis 93° C.

### Beispiel 5

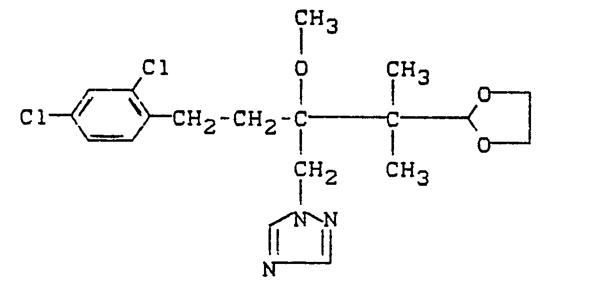

### (Verfahren d / Etherbildung)

Zu 18 g (0,045 Mol) 1-(2,4-Dichlorphenyl)-4-(1,3-dioxan-2-yl)-4-methyl-3-(1,2,4-triazol-1-yl-methyl)-3-pentanol in 150 ml absolutem Dioxan werden 1,4 g (0,046 Mol) Natriumhydrid zugegeben. Man läßt 3 Stunden bei Raumtemperatur nachrühren und versetzt mit 7,1 g (0,05 Mol) Methyliodid. Danach wird über Nacht nachgerührt und erneut mit 0,7 g (0,023 Mol) Natriumhydrid und 3,5 g (0,025 Mol) Methyliodid versetzt. Nach nochmaligem Nachrühren wird von den anorganischen Salzen abgesaugt. Das Filtrat wird eingeengt, der ölige Rückstand in Methylenchlorid aufgenommen, zweimal mit je 600 ml Wasser nachgewaschen, über Natriumsulfat getrocknet und eingeengt. Der Rückstand wird aus Isopropylether umkristallisiert.

Man erhält 12 g (64,4 % der Theorie) 1-(2,4-Dichlorphenyl)-4-(1,3-Dioxan-2-yl)-3-methoxy-4-methyl-3-(1,2,4-triazol-1-yl-methyl)-pentan vom Schmelzpunkt 130°C bis 132°C.

In analoger Weise und entsprechend den erfindungsgemäßen Verfahrensangaben werden die nachfolgenden Verbindungen der allgemeinen Formel (la) erhalten:

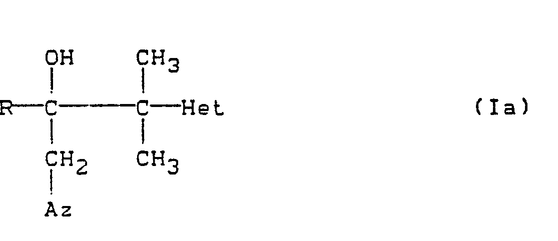

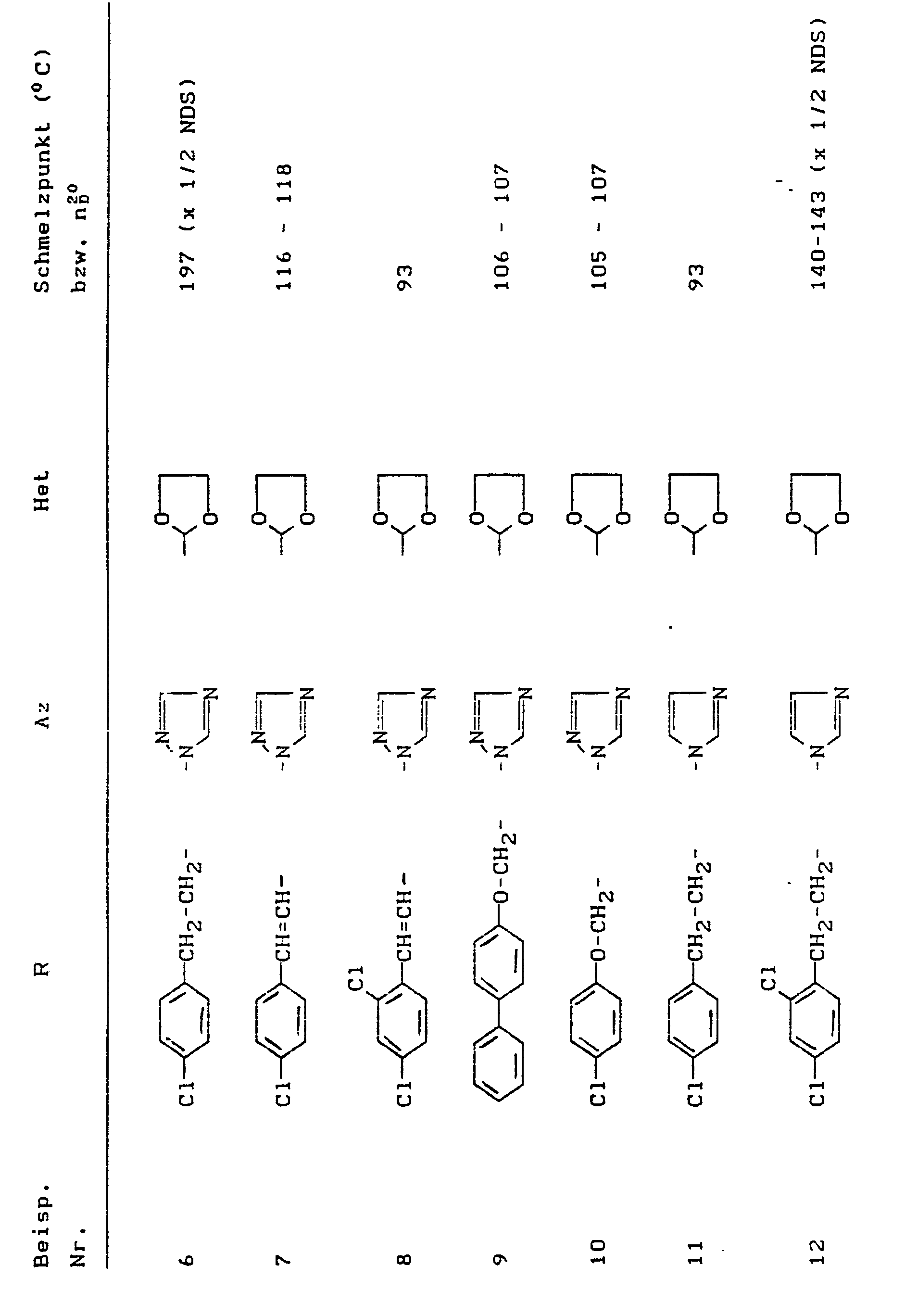

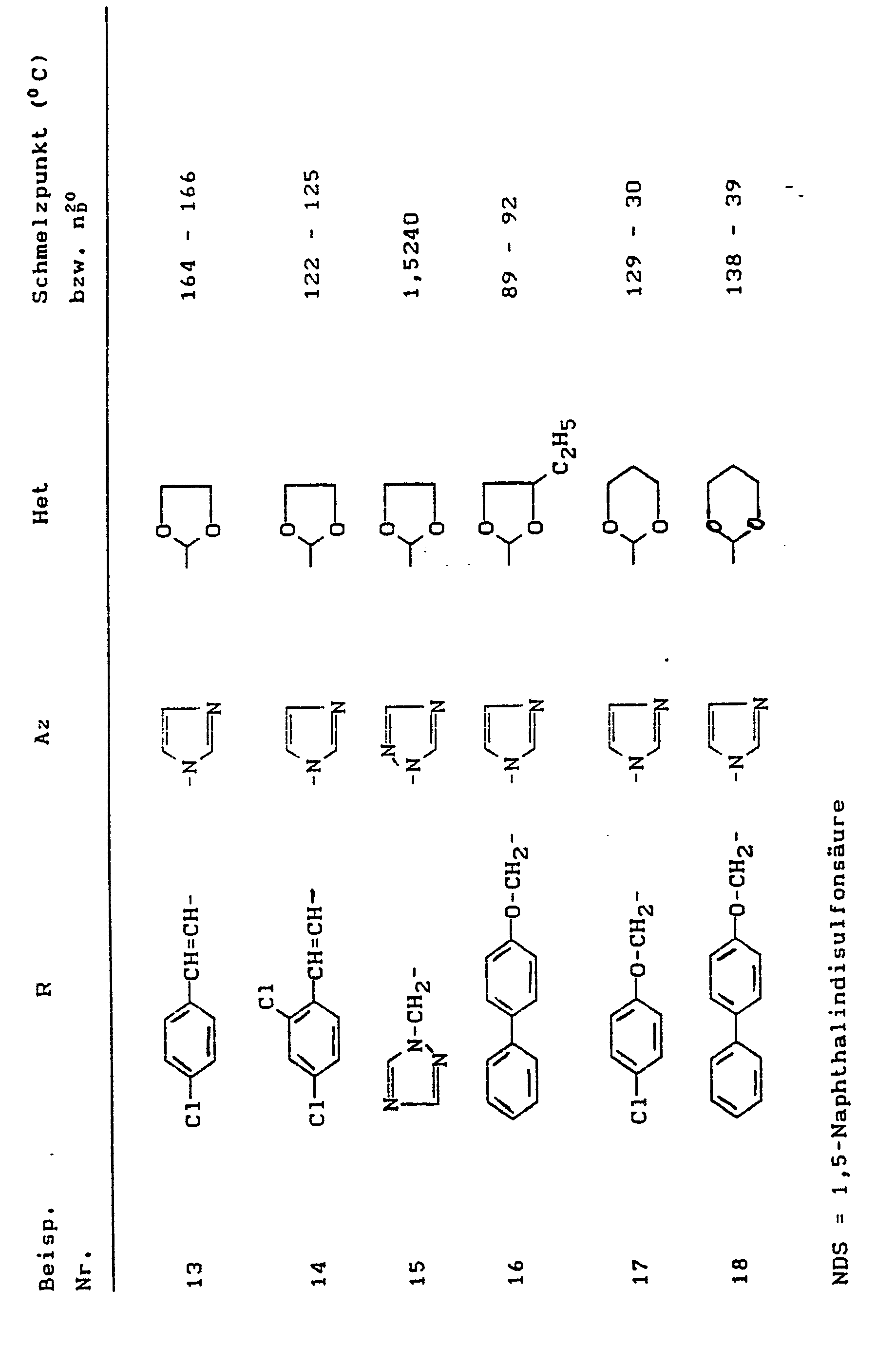

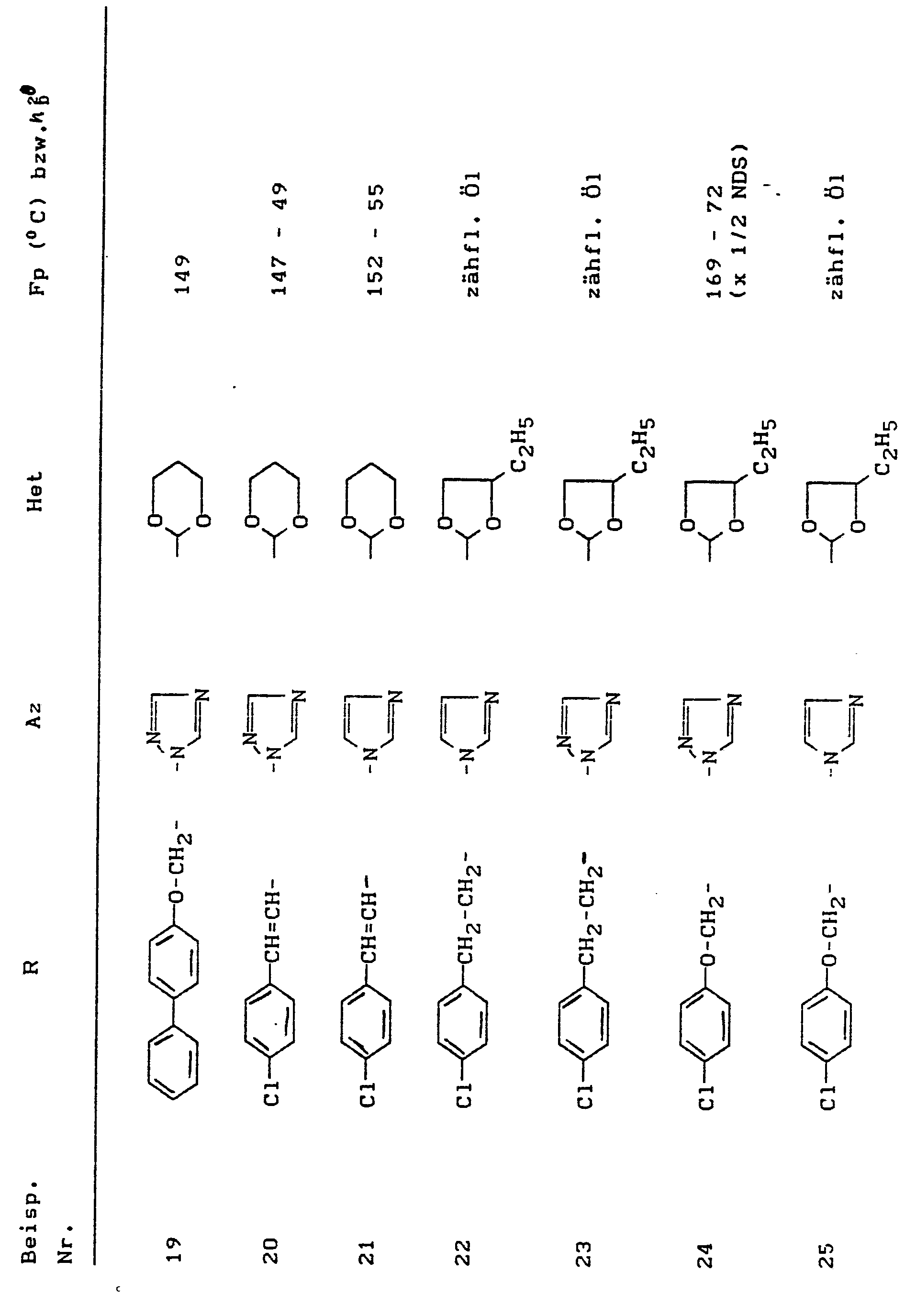

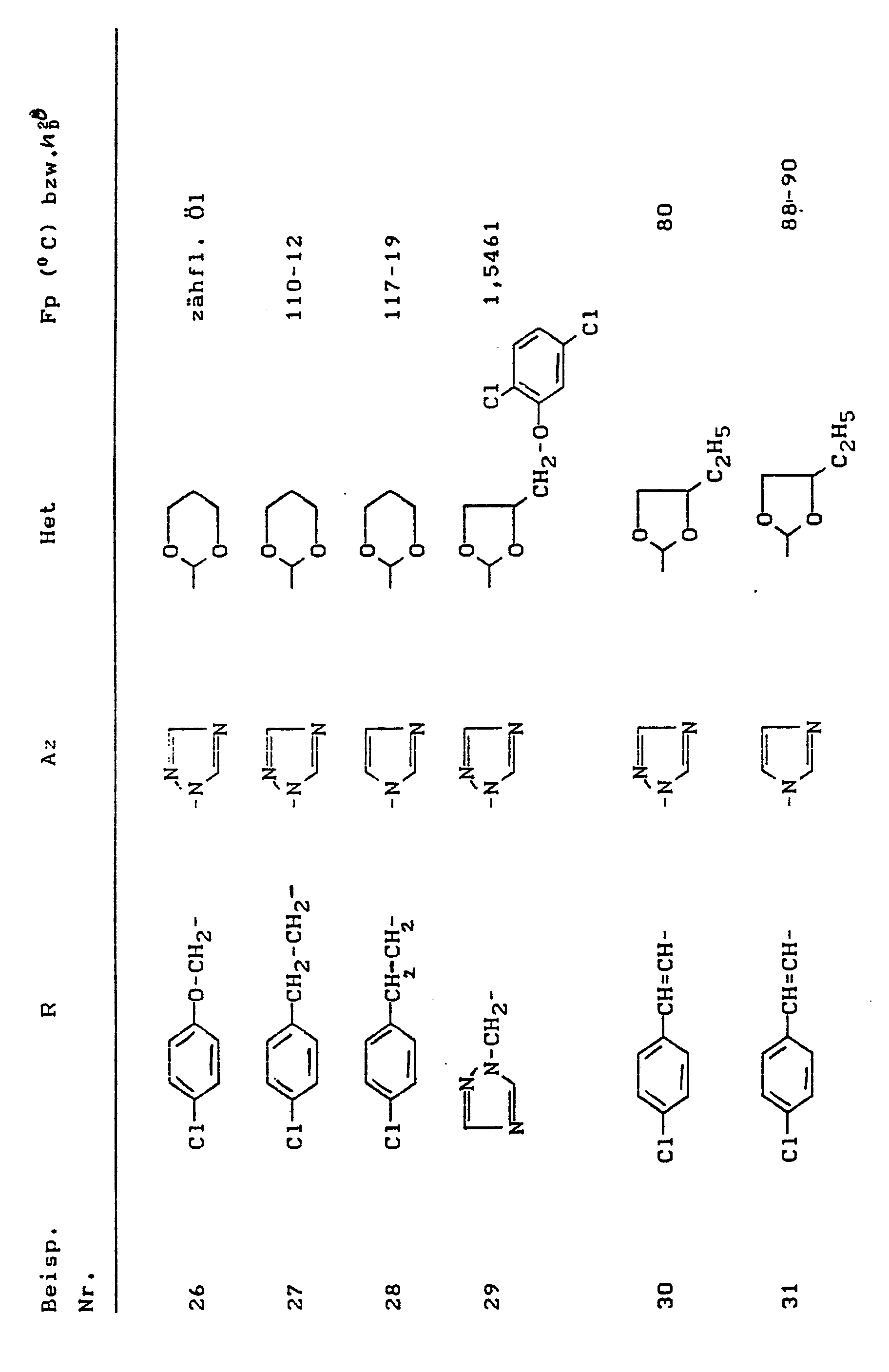

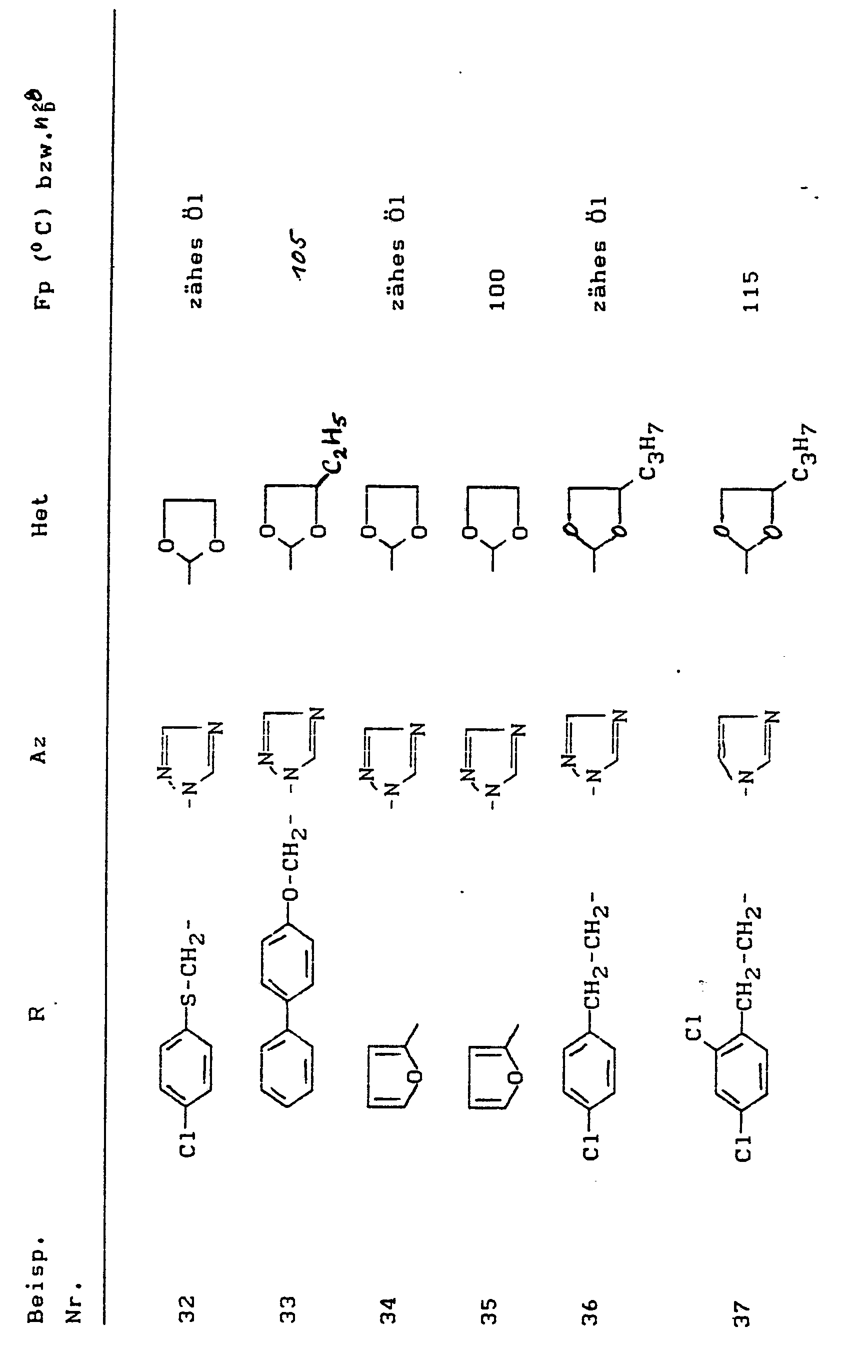

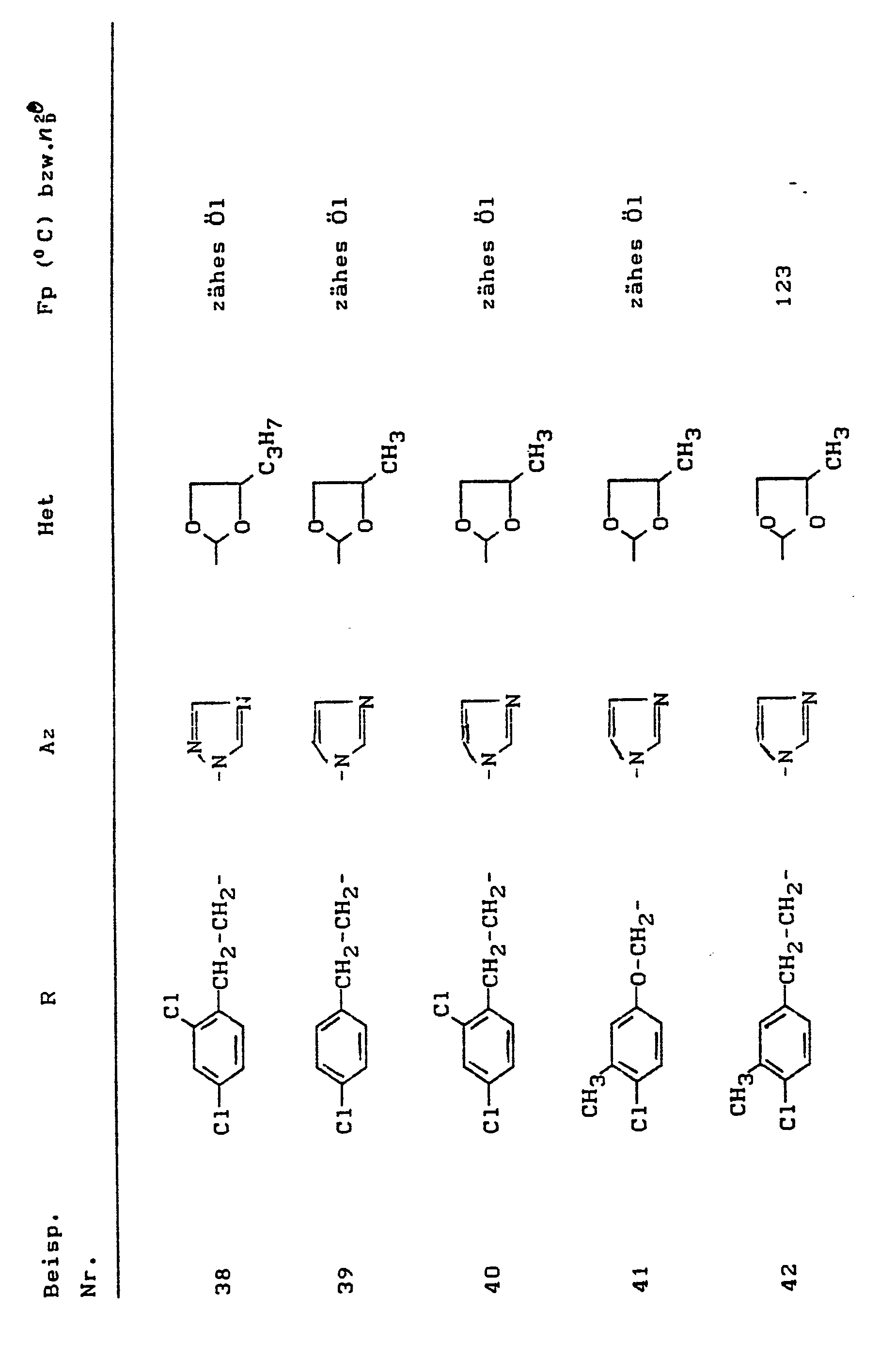

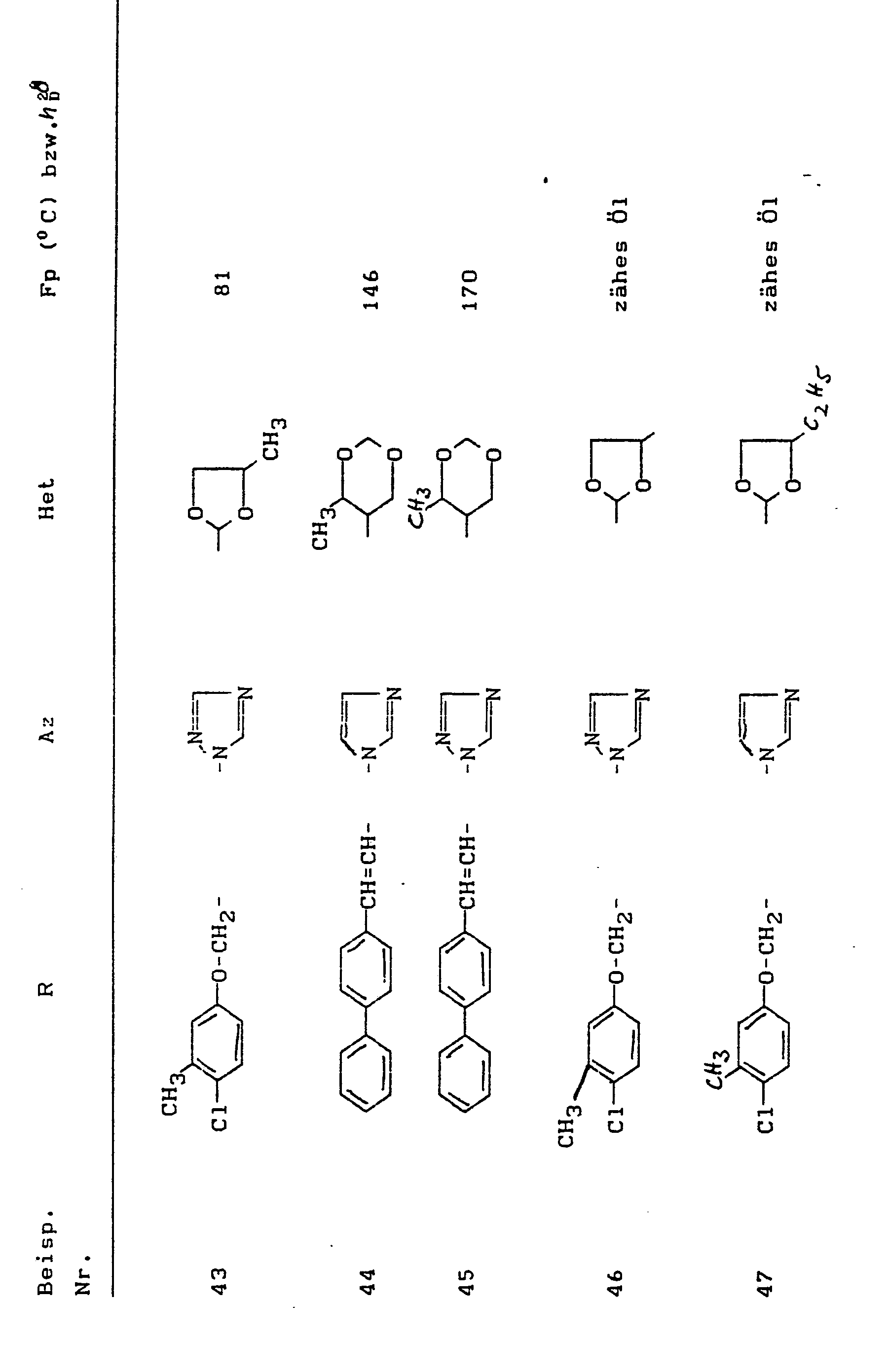

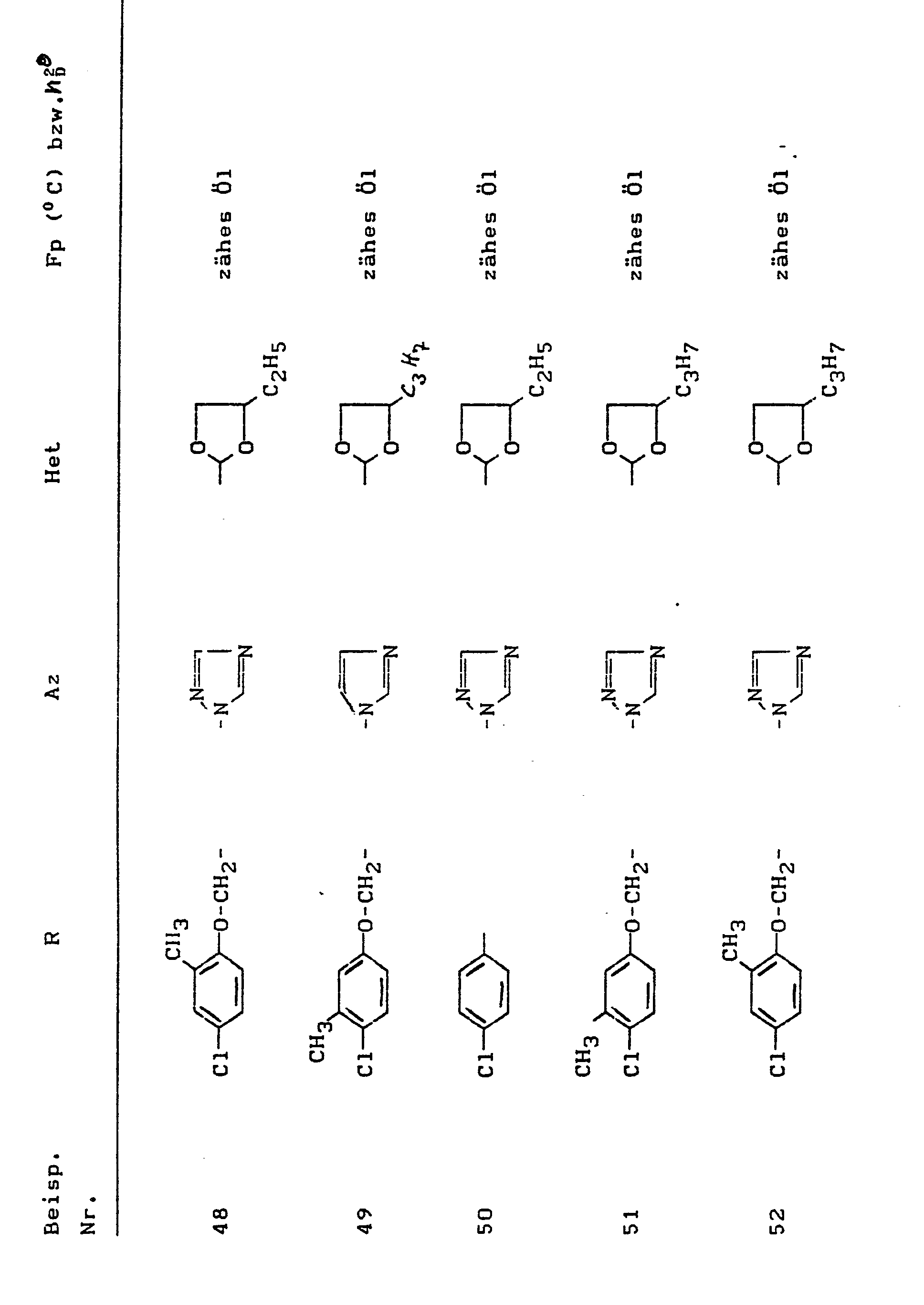

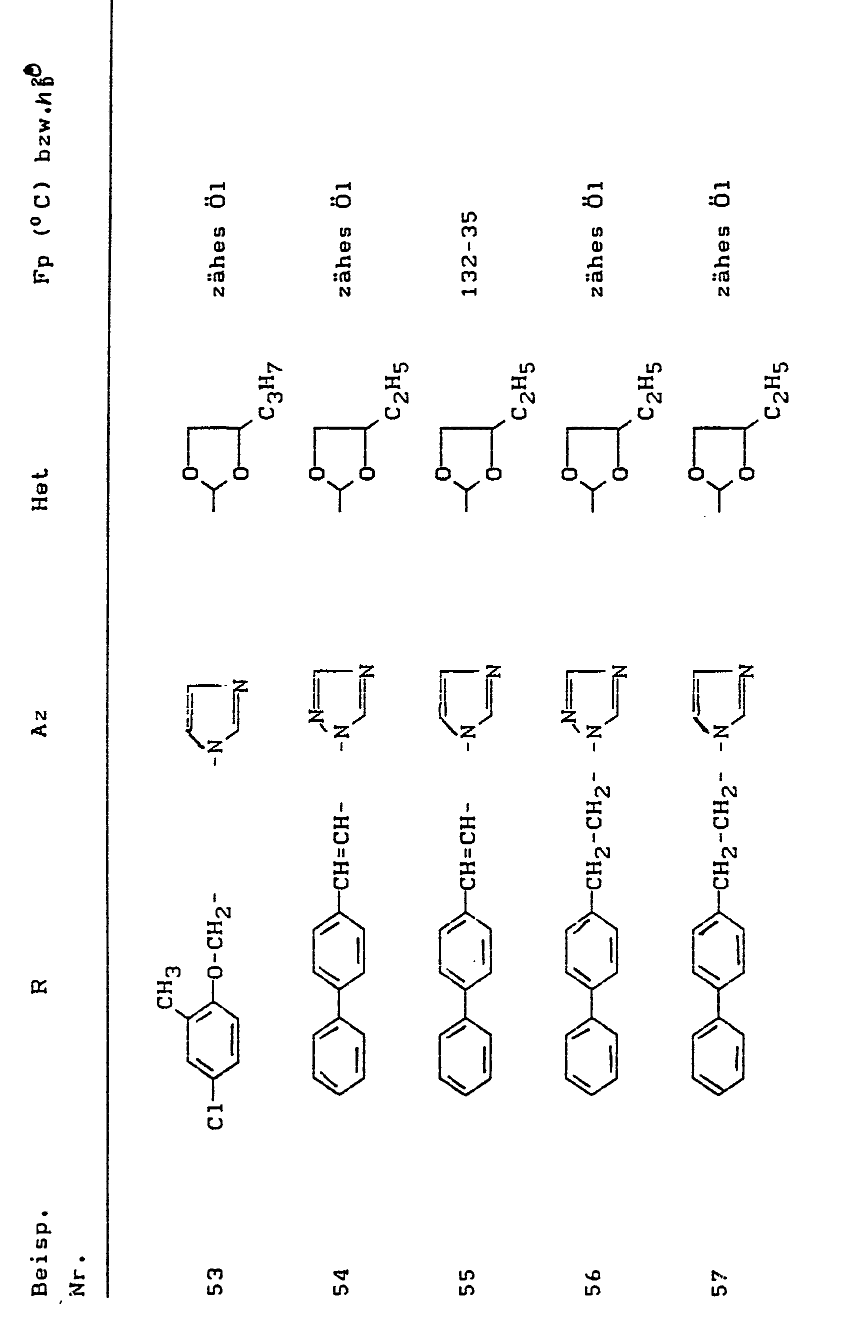

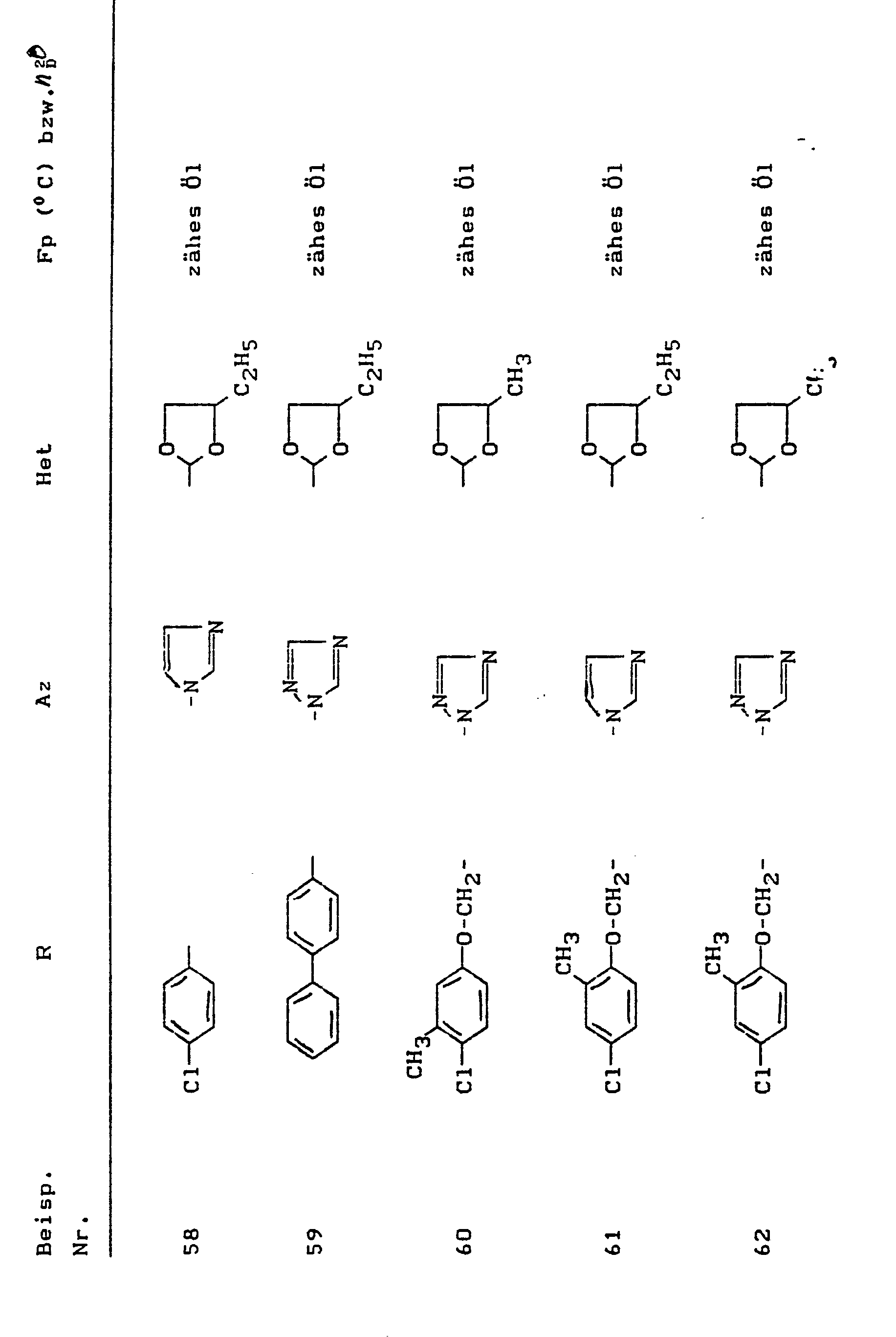

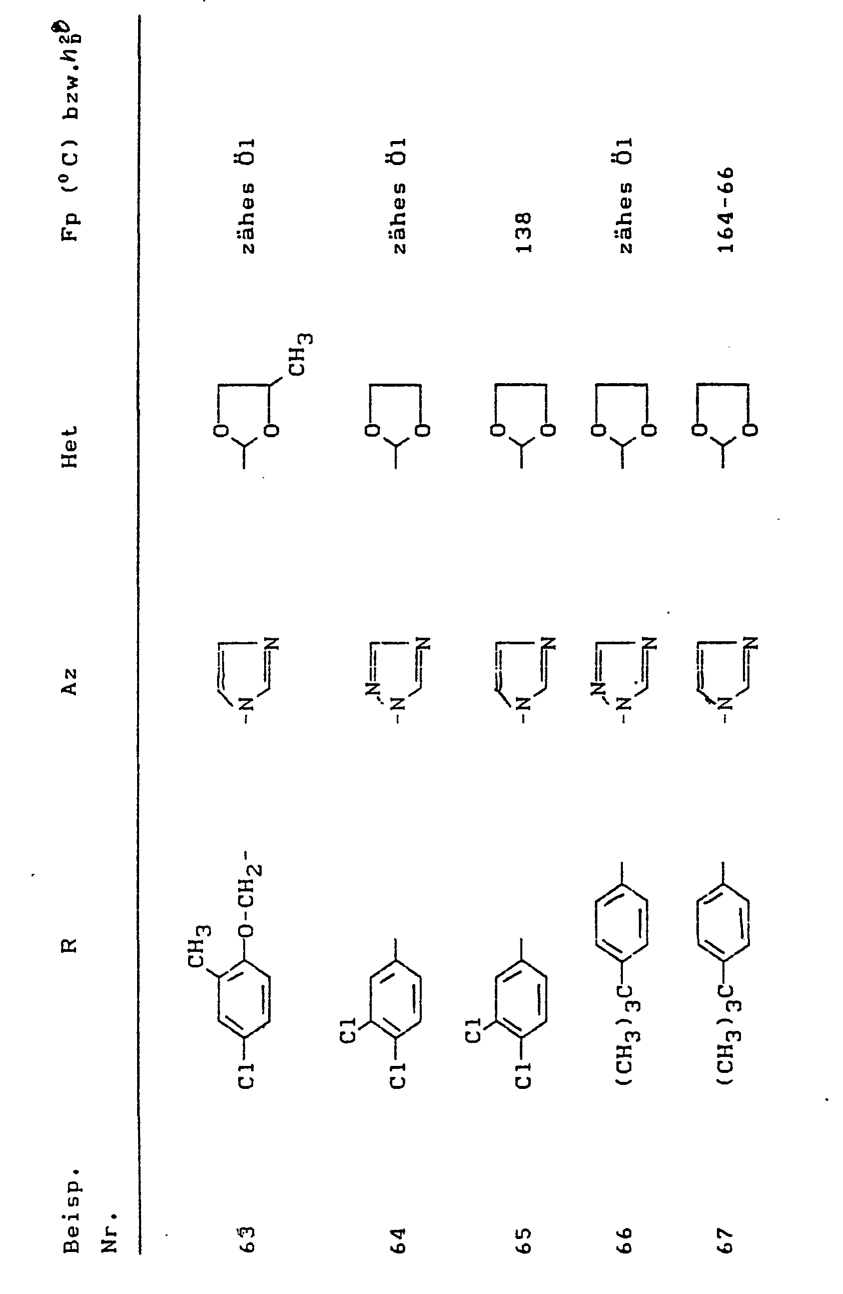

### Verwendungsbeispiele:

In den nachfolgenden Verwendungsbeispielen werden die nachstehend angegebenen Substanzen als Vergleichsverbindungen eingesetzt:

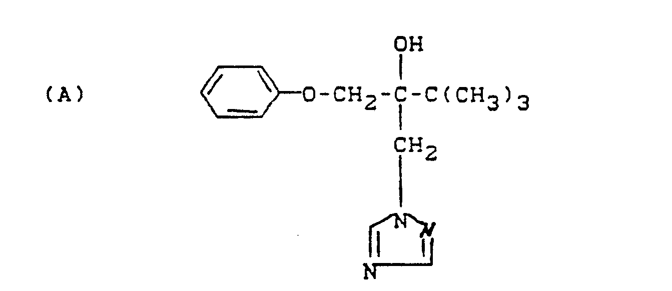

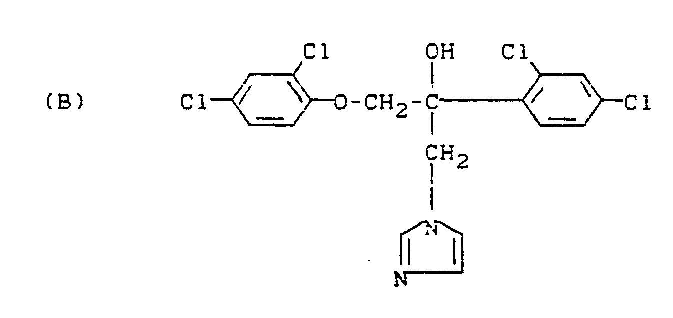

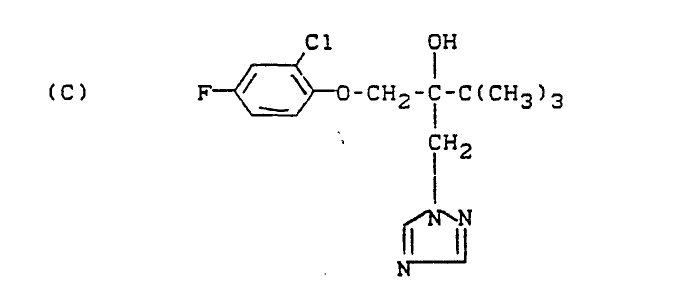

### Beispiel A

Puccinia-Test (Weizen) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita mit in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 7, 13, 8, 14, 6, 11, 4, 12 und 9.

### Beispiel B

Botrytis-Test (Bohne)/protektiv
Lösungsmittel: 4,7 Gewichtsteile Aceton Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß folgender Herstellungsbeispiele: 8, 14, 6, 11 und 4.

## Patentansprüche

1. Heterocyclisch substituierte Hydroxyalkyl-azolylderivate der allgemeinen Formel 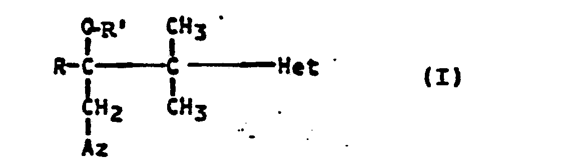 in welcher
Az für 1,2,4-Triazol-1-yl, 1,2,4-Triazol-4-yl oder Imidazol-1-yl steht,
Het für jeweils gegebenenfalls einfach oder mehrfach, gleich oder verschieden substituiertes Dioxolan-2-yl oder 1,3-Dioxanyl steht, wobei als Substituenten zu nennen sind: Alkyl mit 1 bis 4 Kohlenstoffatomen, sowie jeweils gegebenenfalls einfach oder mehrfach, gleich oder verschieden substituiertes Phenyl und Phenoxyalkyl mit 1 bis 4 Kohlenstoffatomen im Alkylteil, wobei als Phenylsubstituenten zu nennen sind: Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen;
R für geradkettiges oder verzweigtes Alkyl mit 1 bis 7 Kohlenstoffatomen steht, ferner für jeweils gegebenenfalls einfach oder mehrfach, gleich oder verschieden substituiertes Phenyl, Phenylalkyl mit 1 bis 4 Kohlenstoffatomen im Alkylteil, Phenoxy- und Phenylthioalkyl mit jeweils 1 bis 4 Kohlenstoffatomen im Alkylteil oder für Phenylethenyl steht, wobei als Substituenten an den Phenyl-Gruppen genannt werden: Halogen, Alkyl mit 1 bis 4 Kohlenstoffatomen,
Alkoxy und Alkylthio mit jeweils 1 bis 2 Kohlenstoffatomen, Nitro, Halogenalkyl und Halogenalkoxy sowie Halogenalkylthio mit jeweils 1 bis 2 Kohlenstoffatomen und 1 bis 5 gleichen oder verschiedenen Halogenatomen, Hydroximirioalkyl mit 1 bis 4 Kohlenstoffatomen, Alkoximinoalkyl mit 1 bis 4 Kohlenstoffatomen in jedem Alkylteil, sowie jeweils gegebenenfalls durch Halogen und Alkyl mit 1 bis 2 Kohlenstoffatomen substituiertes Phenyl, Phenoxy, Benzyl und Benzyloxy;
und
R weiterhin für jeweils gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen steht, für Cycloalkylmethyl oder -ethyl mit 5 bis 7 Kohlenstoffatomen im Cycloalkylteil und Cyclohexylethenyl, schließlich für Alkenyl mit 2 bis 6 Kohlenstoffatomen, für 2-Furyl, für 1,2,4-Triazol-1-yl-methyl, 1,2,4-Triazol-4-yl-methyl, Imidazol-1-yl-methyl und Pyrazol-1-yl-methyl steht,
R' für Wasserstoff, für gegebenenfälls durch Phenyl substituiertes Alkyl mit 1 bis 4 Kohlenstoffatomen, wobei der Phenylrest durch die bei R genannten Phenylsubstitienten substituiert sein kann, sowie für Alkenyl mit 2 bis 4 Kohlenstoffatomen steht, sowie deren Säureadditions-Salze und Metallsalz-Komplexe.

2. Verbindungen der allgemeinen Formel (I) gemäß Anspruch 1, in denen
Het für jeweils gegebenenfalls einfach bis dreifach, gleich oder verschieden substituiertes Dioxolan-2-yl, 1,3-Dioxan-5-yl oder 1,3-Dioxan-2-yl steht, wobei als Substituenten zu nennen sind: Methyl, Ethyl, n-Propyl, Isopropyl sowie jeweils gegebenenfalls einfach bis dreifach, gleich oder verschieden durch Fluor, Chlor, Methyl, Trifluormethyl oder Trifluormethoxy substituiertem Phenyl und Phenoxymethyl;
R für tert.-Butyl, Trimethyl-propyl, Tetramethyl-propyl sowie für jeweils gegebenenfalls einfach bis dreifach, gleich oder verschieden substituiertes Phenyl, Benzyl, Phenethyl, Phenoxymethyl, Phenylthiomethyl oder Phenethenyl steht, wobei als Phenylsubstituenten zu nennen sind: Fluor, Chlor, Methyl, Trifluormethyl, Trifluormethoxy, Trifluormethylthio, Hydroximinomethyl, 1-Hydroximinoethyl, Methoximinomethyl und 1-Methoximinoethyl sowie jeweils gegebenenfalls durch Chlor und/oder Methyl substituiertes Phenyl, Phenoxy, Benzyl und Benzyloxy; R ferner für jeweils gegebenenfalls einfach bis zweifach, gleich oder verschieden durch Methyl, Ethyl oder Isopropyl substituiertes Cyclohexyl, Cyclohexylmethyl, Cyclohexylethyl oder Cycloehexylethenyl steht; R weiterhin für Allyl, Dimethylpropenyl, 2-Furyl, 1,2,4-Triazol-1-yl-methyl, 1,2,4-Triazol-4-yl-methyl, Imidazol-1-yl-methyl oder Pyrazol-1-yl-methyl steht;
R' für Wasserstoff, Methyl, 4-Chlorbenzyl oder Alkyl steht und
Az für 1,2,4 Triazol-1-yl 1,2,4-Triazol-4-yl oder Imidazol-1-yl steht.

3. Verfahren zur Herstellung von heterocyclisch substituierten Hydroxyalkyl-azolyl-Derivaten der allgemeinen Formel 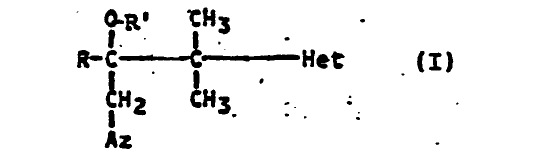 in welcher
Az für 1,2,4-Triazol-1-yl, 1,2,4-Triazol-4-yl oder Imidazol-1-yl steht,
Het für jeweils gegebenenfalls einfach oder mehrfach, gleich oder verschieden substituiertes Dioxolan-2-yl oder 1,3-Dioxanyl steht, wobei als Substituenten zu nennen sind: Alkyl mit 1 bis 4 Kohlenstoffatomen, sowie jeweils gegebenenfalls einfach oder mehrfach, gleich oder verschieden substituiertes Phenyl und Phenoxyalkyl mit 1 bis 4 Kohlenstoffatomen im Alkylteil, wobei als Phenylsubstituenten zu nennen sind: Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen;
R für geradkettiges oder verzweigtes Alkyl mit 1 bis 7 Kohlenstoffatomen steht, ferner für jeweils gegebenfalls einfach oder mehrfach gleich oder verschieden substituiertes Phenyl, Phenylalkyl mit 1 bis 4 Kohlenstoffatomen im Alkylteil, Phenoxy- und Phenylthioalkyl mit jeweils 1 bis 4 Kohlenstoffatomen im Alkylteil oder für Phenylethenyl steht, wobei als Substituenten an den Phenyl-Gruppen genannt werden: Halogen, Alkyl mit 1 bis 4 Kohlenstoffatomen,
Alkoxy und Alkylthio mit jeweils 1 bis 2 Kohlenstoffatomen, Nitro, Halogenalkyl und Halogenalkoxy sowie Halogenalkylthio mit jeweils 1 bis 2 Kohlenstoffatomen und 1 bis 5 gleichen oder verschiedenen Halogenatomen, Hydroximinoalkyl mit 1 bis 4 Kohlenstoffatomen, Alkoximinoalkyl mit 1 bis 4 Kohlehstoffatomen in jedem Alkylteil, sowie jeweils gegebenenfalls durch Halogen und Alkyl mit 1 bis 2 Kohlenstoffatomen substituiertes Phenyl, Phenoxy, Benzyl und Benzyloxy;
und
R weiterhin für jeweils gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen steht, für Cycloalkylmethyl oder -ethyl mit 5 bis 7 Kohlenstoffatomen im Cycloalkylteil und Cyclohexylethenyl, schließlich für Alkenyl mit 2 bis 6 Kohlenstoffatomen, für 2-Furyl, für 1,2,4-Triazol-1-yl-methyl, 1,2,4-Triazol-4-yl-methyl, Imidazol-1-yl-methyl und Pyrazol-1-yl-methyl steht,
R' für Wasserstoff, für gegebenenfalls durch Phenyl substituiertes Alkyl mit 1 bis 4 Kohlenstoffatomen, wobei der Phenylrest durch die bei R genannten Phenylsubstitienten substituiert sein kann, sowie für Alkenyl mit 2 bis 4 Kohlenstoffatomen steht, sowie deren Säureadditions-Salzen und Metallsalz-Komplexen, dadurch gekennzeichnet, daß man
a) Oxirane der Formel 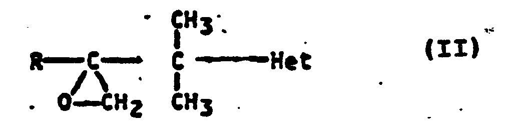 in welcher
Het und R die oben angegebene Bedeutung haben,
mit Azolen der Formel (111) 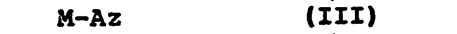 in welcher
Az die oben angegebene Bedeutung hat und
M für Wasserstoff oder ein Alkalimetallsälz steht,
in Gegenwart eines Verdünnungsmittels und gegebenenfalls in Gegenwärt einer Base umsetzt, oder
b) Azoloketone der Formel (IV) 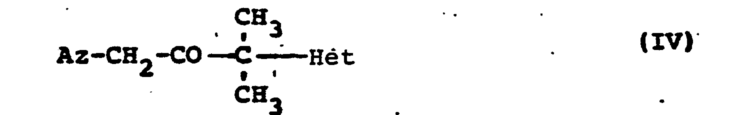 in welcher
Az und Het die oben angegebene Bedeutung haben,
mit einer magnesium-organischen Verbindung der Formel (V) 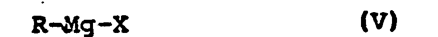 in welcher
R die oben angegebene Bedeutung hat und
X für Chlor, Brom oder Jod steht,
in Gegenwart eines Verdünnungsmittels umsetzt,
oder
c) Azolooxirane der Formel (VI) 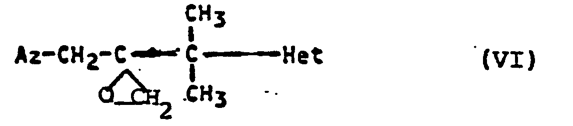 in welcher
Az und Het die oben angegebene Bedeutung haben, mit gegebenenfalls substituierten Phenolen und Thiophenolen sowie mit metallorganischen Verbindungen der Formel (VII) 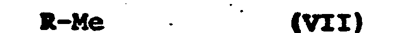 in welcher
R die oben angegebene Bedeutung hat und
Me für ein Alkalimetall oder -Mg-X steht, wobei X die oben angegebene Bedeutung hat,
in Gegenwart eines Verdünnungsmittels und gegebenenfalls in Gegenwart einer Base umsetzt, und gegbenenfalls noch
d) die nach den Verfahren a), b) oder c) erhaltenen Hydroxy-Verbindungen in Gegenwart eines Verdünnungsmittels in das Alkalimetall-alkoholat überführt und dieses mit einem Halogenid zu den entsprechenden Ether-Derivaten umsetzt,
und gegebenenfalls noch an die so erhaltenen Verbindungen der Formel (I) anschließend eine Säure oder ein Metallsalz addiert.

4. Fungizide Mittel, gekennzeichnet durch einen Gehalt an mindestens einem heterocyclisch substituierten Hydroxyalkyl-azolyl-Derivat der Formel (I) in Anspruch 1 bzw. einem Säureadditions-Salz oder Metallsalz-Komplex einer Verbindung der Formel (I).

5. Verwendung von heterocyclisch substituierten Hydroxyalkylazolyl-Derivaten der Formel (I) in Anspruch 1 bzw. von deren Säureaddditions-Salzen oder Metallsalz-Komplexen als Pflanzenschutzmittel.

6. Verwendung von heterocyclisch substituierten Hydroxyalkyl-azolyl-Derivaten der Formel (I) in Anspruch 1 bzw. von deren Säureadditions-Salzen oder Metallsalz-Komplexen zur Bekämpfung von Pilzen.

7. Verfahren zur Herstellung von fungiziden Mitteln, dadurch gekennzeichnet, daß man heterocyclisch substituierte Hydroxyalkyl-azolyl-Derivate der Formel (I) in Anspruch 1 bzw. deren Säureadditions-Salze oder Metallsalz-Komplexe mit Streckmitteln und/oder oberflächenaktiven Mitteln vermischt.

## Claims

1. Hydroxyalkyl azolyl derivatives substituted by heterocyclic substituents, of the general formula 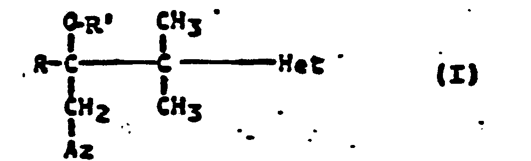 in which
Az represents 1,24-triazoI-1-yI, 1,2,4-triazol-4-yl or imidazol-1-yl,
Het represents dioxolan-2-yl or 1,3-dioxanyl each of which is optionally monosubstituted or polysubstituted by identical or different substituents, the following to be mentioned as substituents: alkyl with 1 to 4 carbon atoms, and phenyl and phenoxyalkyl with 1 to 4 carbon atoms in the alkyl part, each of which is optionally monosubstituted or polysubstituted by identical or different substituents, the following to be mentioned as phenyl substituents: halogen, alkyl with 1 to 4 carbon atoms, alkoxy and alkylthio with in each case 1 to 2 carbon atoms and halogenoalkyl, halogenoalkoxy and halogenoalkylthio with in each case 1 to 2 carbon atoms and 1 to 5 identical or different halogen atoms;
R represents straight-chain or branched alkyl with 1 to 7 carbon atoms, and also phenyl, phenylalkyl with 1 to 4 carbon atoms in the alkyl part, phenoxy- and phenylthioalkyl with in each case 1 to 4 carbon atoms in the alkyl part or phenylethenyl, each of which is optionally monosubstituted or polysubstituted by identical or different substituents, the following being mentioned as substituents on the phenyl groups: halogen, alkyl with 1 to 4 carbon atoms, alkoxy and alkylthio with in each case 1 to 2 carbon atoms; nitro, halogenoalkyl and halogenoalkoxy and halogenoalkylthio with in each case 1 to 2 carbon atoms and 1 to 5 identical or different halogen atoms, hydroximinoalkyl with 1 to 4 carbon atoms, alkoximinoalkyl with 1 to 4 carbon atoms in each alkyi part, and phenyl, phenoxy, benzyl and benzyloxy, each of which is optionally substituted by halogen and alkyl with 1 to 2 carbon atoms; and R furthermore represents cycloalkyl with 5 to 7 carbon atoms, which is in each case optionally monosubstituted or polysubstituted by identical or different alkyl radicals with 1 to 4 carbon atoms, and cycloalkylmethyl or -ethyl with 5 to 7 carbon atoms in the cycloalkyl part, and cyclohexylethenyl, and, finally, alkenyl with 2 to 6 carbon atoms, 2-furyl, 1,2,4-triazol-1-yl-methyl, 1,2,4-triazol-4-yl-methyl, imidazol-1-yl-methyl and pyrazol-1-yl-methyl,
R' represents hydrogen, alkyl which has 1 to 4 carbon atoms and is optionally substituted by phenyl, it being possible for the phenyl radical to be substituted by the phenyl substituents mentioned under R, and alkenyl with 2 to 4 carbon atoms,
and acid addition salts and metal salt complexes thereof.

2. Compounds of the general formula (I) according to Claim 1 in which
Het represents dioxolan-2-yl, 1,3-dioxan-5-yl or 1,3-dioxan-2-yl, each of which is optionally mono- to tri- substituted by identical or different substituents, the following to be mentioned as substituents: methyl, ethyl, n-propyl, isopropyl and phenyl and phenoxymethyl, each of which is optionally mono- to tri-substituted by identical or different substituents from the group comprising fluorine, chlorine, methyl, trifluoromethyl and trifluoromethoxy;
R represents tert.-butyl; trimethyl-propyl or tetramethyl-propyl, and phenyl, benzyl, phenethyl, phenoxymethyl, phenylthiomethyl or phenethenyl, each of which is optionally mono- to trisubstituted by identical or different substituents, the following to be mentioned as phenyl substituents: fluorine, chlorine, methyl, trifluoromethyl, trifluoromethoxy, trifluoromethylthio, hydroximinomethyl, 1-hydroximinoethyl, methoximinomethyl and 1-methoximinoethyl, and phenyl, phenoxy, benzyl and benzyloxy, each of which is optionally substituted by chlorine and/or methyl; R also represents cyclohexyl, cyclohexylmethyl, cyclohexylethyl or cyclohexylethenyl, each of which is optionally mono- to di-substituted by identical or different substituents from the group comprising methyl, ethyl and isopropyl. R furthermore represents allyl, dimethylpropenyl, 2-furyl, 1,2,4-triazol-1-yl-methyl, 1,2,4-triazol-4-yl-methyl, imidazol-1-yl-methyl or pyrazol-1-yl-methyl;
R' represents hydrogen, methyl, 4-chlorobenzyl or alkyl, and
Az represents 1,2,4-triazol-1-y1,1,2,4-triazol-4-yl or imidazol-1-yl.

3. Process for the preparation of hydroxyalkyl-azolyl derivatives substituted by heterocyclic substituents, of the general formula 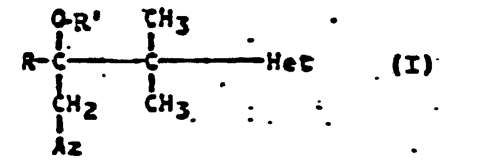 in which
Az represents 1,2,4-triazol-1 -yi, 1,2,4-triazol-4-yl or imidazol-1-yl,
Het represents dioxolan-2-yl or 1,3-dioxanyl, each of which is optionally monosubstituted or polysubstituted by identical or different substituents, the following to be mentioned as substituents: alkyl with 1 to 4 carbon atoms, and phenyl and phenoxyalkyl with 1 to 4 carbon atoms in the alkyl part, each of which is optionally monosubstituted or polysubstituted by identical or different substituents, the following to be mentioned as phenyl substituents: halogen, alkyl with 1 to 4 carbon atoms, alkoxy and alkylthio with in each case 1 to 2 carbon atoms and halogenoalkyl, halogenoalkoxy and halogenoalkylthio with in each case 1 to 2 carbon atoms and 1 to 5 identical or different halogen atoms;
R represents straight-chain or branched alkyl with 1 to 7 carbon atoms, and also phenyl, phenylalkyl with 1 to 4 carbon atoms in the alkyl part, phenoxy- and phenylthioalkyl with in each case 1 to 4 carbon atoms in the alkyl part or phenylethenyl, each of which is optionally monosubstituted or polysubstituted by identical or different substituents, the following being mentioned as substituents on the phenyl groups: halogen alkyl with-1 to 4 carbon atoms, alkoxy and alkylthio with in each case 1 to 2 carbon atoms. nitro, halogenoalkyl and halogencalkoxy and halogenoalkylthio with in each case 1 to 2 carbon atoms and 1 to 5 identical or different halogen atoms, hydroximinoalkyl with 1 to 4 carbon atoms, alkoximinoalkyl with 1 to 4 carbon atoms in each alkyl part, and phenyl, phenoxy, benzyl and benzyloxy, each of which is optionally substituted by halogen and alkyl with 1 to 2 carbon atoms; and R furthermore represents cycloalkyl with 5 to 7 carbon atoms, which is in each case optionally monosubstituted or polysubstituted by identical or different alkyl radicals with 1 to 4 carbon atoms, and cycloalkylmethyl or -ethyl with 5 to 7 carbon atoms in the cycloalkyl part, and cyclohexylethenyl, and, finally, alkenyl with 2 to 6 carbon atoms, 2-furyl, 1,2,4-triazol-1-yi-methyl, 1,2,4-triazol-4-yl-methyl, imidazol-1-yl-methyl and pyrazol-1-yl-methyl,
R' represents hydrogen, alkyl which has 1 to 4 carbon atoms and is optionally substituted by phenyl, it being possible for the phenyl radical to be substituted by the phenyl substituents mentioned under R, and alkenyl with 2 to 4 carbon atoms,
and acid addition salts and metal salt complexes thereof characterised in that
a) oxiranes of the formula (II) 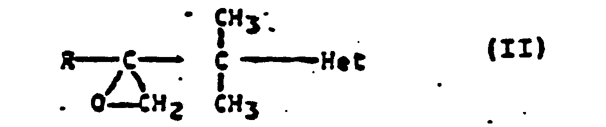 in which
Het and R have the abovementioned meaning, are reacted with azoles of the formula (III) 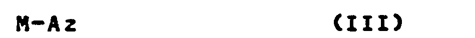 in which
Az has the abovementioned meaning and
M represents hydrogen or an alkali metal salt, in the presence of a diluent and, if appropriate, in the presence of a base, or
b) azolo-ketones of the formula (IV) 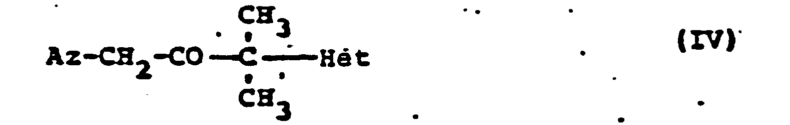 in which
Az and Het have the abovementioned meaning, are reacted with an organomagnesium compound of the forformula (V) 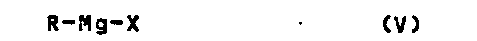 in which
R has the abovementioned meaning and
X represents chlorine, bromine or iodine, in the presence of a diluent, or
c) azolo-oxiranes of the formula (VI) 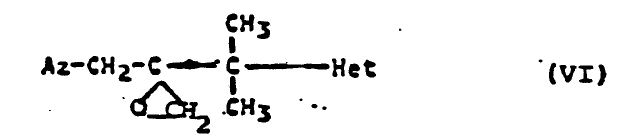 in which
Az and Het have the abovementioned meaning, are reacted with optionally substituted phenols and thiophenols and with organometallic compounds of the formula (VII) 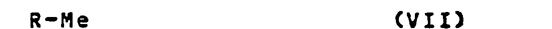 in which
R has the abovementioned meaning and
Me represents an alkali metal or -Mg-X, wherein
X has the abovementioned meaning,
in the presence of a diluent and if appropriate in the presence of a base, and subsequently, if appropriate,
d) the hydroxy compounds obtained according to processes a), b) or c) are converted into the alkali metal alcoholate in the presence of a diluent, and this product is reacted with a halide to give the corresponding ether derivatives, and, if desired, an acid or a metal salt is then subsequently added on to the compounds of the formula (I) thus obtained.

4. Fungicidal agents, characterised in that they contain at least one hydroxyalkyl-azolyl derivative substituted by heterocyclic substituents, of the formula in Claim 1 or an acid addition salt or metal salt complex of a compound of the formula (I).

5. Use of hydroxyalkyl-azolyl derivatives substituted by heterocyclic substituents, of the formula (I) in Claim 1, or acid addition salts or metal salt complexes thereof as plant protection agents.

6. Use of hydroxyalkyl-azolyl derivatives substituted by heterocyclic substituents, of the formula (I) in Claim 1, or acid addition salts or metal salt complexes thereof for combating fungi.

7. Process for the preparation of fungicidal agents, characterised in that hydroxyalkyl-azolyl derivatives substituted by heterocyclic substituents, of the formula (I) in Claim 1, or acid addition salts or metal salt complexes thereof are mixed with extenders and/or surface-active agents.

## Revendications

1. Dérivés hydroxyalkylazolyliques à substituant hétérocyclique, de formule générale 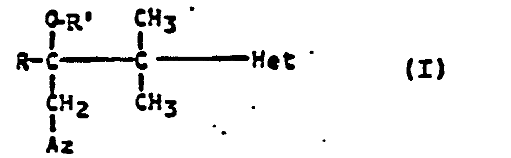 dans laquelle
Az est un groupe 1,2,4-triazole-1-yle, 1,2,4-triazole-4-yle ou imidazole-1-yle,
Het est un groupe dioxolane-2-yle ou un groupe 1,3-dioxanyle dont chacun porte, le cas échéant, un ou plusieurs substituants identiques ou différents, et on mentionne alors comme substituants: un groupe alkyle ayant 1 à 4 atomes de carbone, ainsi qu'un groupe phényle et un groupe phénoxyalkyle ayant 1 à 4 atomes de carbone dans la partie alkyle, chacun portant, le cas échéant, un ou plusieurs substituants identiques ou différents, et on mentionne alors comme substituants du groupe phényle: un halogène, un groupe alkyle ayant 1 à 4 atomes de carbone, un groupe alkoxy et un groupe alkylthio ayant chacun 1 ou 2 atomes de carbone, ainsi qu'un groupe halogénalkyle, un groupe halogénalkoxy et un groupe halogénalkylthio ayant chacun 1 ou 2 atomes de carbone et 1 à 5 atomes d'halogènes identiques ou différents;
R est un groupe alkyle à chaîne droite ou ramifié ayant 1 à 7 atomes de carbone, en outre un groupe phényle, un groupe phénylalkyle ayant 1 à 4 atomes de carbone dans la partie alkyle, un groupe phénoxyalkyle et un groupe phénylthioalkyle ayant chacun 1 à 4 atomes de carbone dans la partie alkyle ou un groupe phényléthényle, portant chacun, le cas échéant, un ou plusieurs substituants identiques ou différents, et on mentionne alors comme substituants des groupes phényle: un halogène, un groupe alkyle ayant 1 à 4 atomes de carbone, un groupe alkoxy et un groupe alkylthio ayant chacun 1 ou 2 atomes de carbone, un groupe nitro, un groupe halogénalkyle et un groupe halogénalkoxy ainsi qu'un groupe halogénalkylthio ayant chacun 1 ou 2 atomes de carbone et 1 à 5 atomes d'halogènes identiques ou différents, un groupe hydroximinoalkyle ayant 1 à 4 atomes de carbone, un groupe alkoximinoalkyle ayant 1 à 4 atomes de carbone dans chaque partie alkyle, ainsi que des groupes phényle, phénoxy, benzyle et benzyloxy substitués dans chaque cas, le cas échéant, par un halogène et un groupe alkyle ayant 1 ou 2 atomes de carbone; et
R désigne en outre un groupe cycloalkyle de 5 à 7 atomes de carbone, un groupe cycloalkylméthyle ou -éthyle de 5 à 7 atomes de carbone dans la partie cycloalkyle et un groupe cyclohexyléthényle portant chacun, le cas échéant, un ou plusieurs substituants alkyle identiques ou différents ayant 1 à 4 atomes de carbone, enfin un groupe alcényle de 2 à 6 atomes de carbone, le groupe 2-furyle les groupes 1,2,4-triazole-1-ylméthyle 1 24-triazole-4-ylméthyle, imidazole-1-ylméthyle et pyrazole-1-ylméthyle,
R' représente l'hydrogène, un groupe alkyle de 1 à 4 atomes de carbone éventuellement substitué par un reste phényle, le reste phényle pouvant être substitué par les substituants du groupe phényle mentionnés pour R, ainsi qu'un groupe alcényle ayant 2 à 4 atomes de carbone,
de même que leurs sels d'addition d'acides et leurs complexes de sels métalliques.

2. Composés de formule générale (I) suivant la revendication 1, dans lesquels
Het est un groupe dioxolane-2-yle, 1,3-dioxane-5-yle ou 1,3-dioxane-2-yle portant chacun, le cas échéant, un à trois substituants identiques ou différents, et on mentionne alors comme substituants: un groupe méthyle, éthyle, n-propyle, isopropyle ainsi qu'un groupe phényle et un groupe phénoxyméthyle portant chacun, le cas échéant, un à trois substituants identiques ou différents tels que fluor, chlore, méthyle, trifluorométhyle ou trifluorométhoxy;
R est un groupe tertio-butyle, triméthylpropyle, tétraméthylpropyle ainsi qu'un groupe phényle benzyle, phénéthyle, phénoxyméthyle, phénylthiométhyle ou phénéthényle portant chacun, le cas échéant, un à trois substituants identiques ou différents, et on mentionne alors comme substituants du reste phényle: le fluor, le chlore, un groupe méthyle, trifluorométhyle, trifluorométhoxy, trifluorométhylthio, hydroximinométhyle, 1-hydroximinoéthyle, méthoximinométhyle et 1-méthoximinoéthyle, ainsi que des groupes phényle, phénoxy, benzyle et benzyloxy substitués chacun, le cas échéant, par du chlore et/ou par un groupe méthyle; R désigne en outre un groupe cyclohexyle, cyclohexylméthyle, cyclohexyléthyle ou cyclohexyléthényle portant chacun, le cas échéant, un ou deux substituants méthyle, éthyle ou isopropyle identiques ou différents; R est en outre un groupe allyle, diméthylpropényle, 2-furyle, 1,2,4-triazole-1-ylméthyle, 1,2,4-triazole-4-ylméthyle, imidazole-1- ylméthyle ou pyrazole-1-ylméthyle;
R' est l'hydrogène ou un groupe méthyle, 4-chlorobenzyle ou alkyle et
Az est un groupe 1,2,4-triazole-1-yle, 1,2,4-triazole-4-yle ou imidazole-1-yle.

3. Procédé de production de dérivés hydroxyalkylazolyliques à substituant hétérocyclique de formule générale 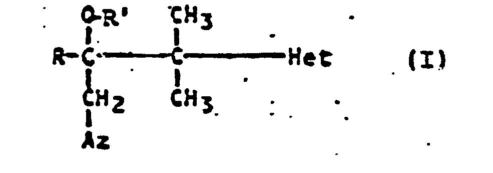 dans laquelle
Az est un groupe 1,2,4-triazole-1-yle, 1,2,4-triazole-4-yle ou imidazole-1-yle,
Het est un groupe dioxolane-2-yle ou un groupe 1,3-dioxanyle dont chacun porte, le cas échéant, un ou plusieurs substituants identiques ou différents, et on mentionne alors comme substituants: un groupe alkyle ayant 1 à 4 atomes de carbone, ainsi qu'un groupe phényle et un groupe phénoxyalkyle ayant 1 à 4 atomes de carbone dans la partie alkyle, chacun portant, le cas échéant, un ou plusieurs substituants identiques ou différents, et on mentionne alors comme substituants du groupe phényle: un halogène, un groupe alkyle ayant 1 à 4 atomes de carbone, un groupe alkoxy et un groupe alkylthio ayant chacun 1 ou 2 atomes de carbone, ainsi qu'un groupe halogénalkyle, un groupe halogénalkoxy et un groupe halogénalkylthio ayant chacun 1 ou 2 atomes de carbone et 1 à 5 atomes d'halogènes identiques ou différents;
R est un groupe alkyle à chaîne droite ou ramifié ayant 1 à 7 atomes de carbone, en outre un groupe phényle, un groupe phénylalkyle ayant 1 à 4 atomes de carbone dans la partie alkyle, un groupe phénoxyalkyle et un groupe phénylthioalkyle ayant chacun 1 à 4 atomes de carbone dans la partie alkyle ou un groupe phényléthényle, portant chacun, le cas échéant, un ou plusieurs substituants identiques ou différents, et on mentionne alors comme substituants des groupes phényle: un halogène, un groupe alkyle ayant 1 à 4 atomes de carbone, un groupe alkoxy et un groupe alkylthio ayant chacun 1 ou 2 atomes de carbone, un groupe nitro, un groupe halogénalkyle et un groupe halogénalkoxy ainsi qu'un groupe halogénalkylthio ayant chacun 1 ou 2 atomes de carbone et 1 à 5 atomes d'halogènes identiques ou différents, un groupe hydroximinoalkyle ayant 1 à 4 atomes de carbone, un groupe alkoximinoalkyle ayant 1 à 4 atomes de carbone dans chaque partie alkyle, ainsi que des groupes phényle, phénoxy, benzyle et benzyloxy substitués dans chaque cas, le cas échéant, par un halogène et un groupe alkyle ayant 1 ou 2 atomes de carbone;
et
R désigne en outre un groupe cycloalkyle de 5 à 7 atomes de carbone, un groupe cycloalkylméthyle ou -éthyle de 5 à 7 atomes de carbone dans la partie cycloalkyle et un groupe cyclohexyléthényle, portant chacun, le cas échéant, un ou plusieurs substituants alkyle identiques ou différents ayant 1 à 4 atomes de carbone, enfin un groupe alcényle de 2 à 6 atomes de carbone, le groupe 2-furyle les groupes 1,2,4-triazole-1-ylméthyle 1,2,4-triazole-4-ylméthyle, imidazole-1-ylméthyle et pyrazole-1-yl-méthyle,
R' représente l'hydrogène, un groupe alkyle de 1 à 4 atomes de carbone éventuellement substitué par un reste phényle, le reste phényle pouvant être substitué par les substituants du groupe phényle mentionnés pour R, ainsi qu'un groupe alcényle ayant 2 à 4 atomes de carbone, ainsi que de leurs sels d'addition d'acides et de leurs complexes de sels métalliques, caractérisé en ce
a) on fait réagir des oxiranes de formule (II) 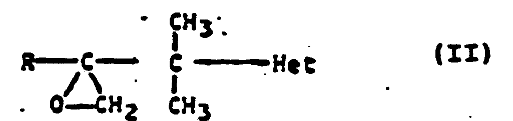 dans laquelle
Het et R ont la définition indiquée ci-dessus, avec des azoles de formule (lll) 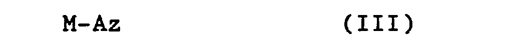 dans laquelle
Az a la définition indiquée ci-dessus et
M désigne l'hydrogène ou un sel de métal alcalin,
en présence d'un diluant et, le cas échéant, en présence d'une base,
ou bien
b) on fait réagir des azolocétones de formule (IV) 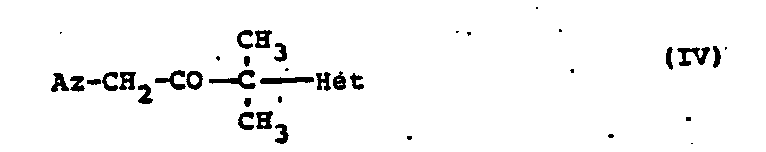 dans laquelle
Az et Het ont la définition indiquée ci-dessus, avec un composé organique du magnésium de formule (V) 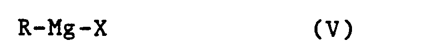 dans laquelle
R a la définition indiquée ci-dessus et
X représente du chlore, du brome ou de l'iode, en présence d'un diluant, ou bien
c) on fait réagir des azolo-oxiranes de formule (VI) 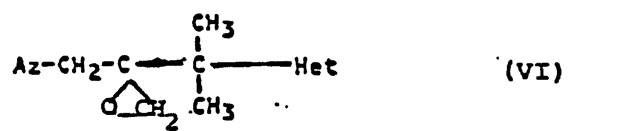 dans laquelle
Az et Het ont la définition indiquée ci-dessus avec des phénols et des thiophénols éventuellement substitués ainsi qu'avec des composés organométalliques de formule (VII) 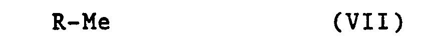 dans laquelle
R a la définition indiquée ci-dessus et
Me représente un métal alcalin ou -Mg-X, où
X a la définition indiquée ci-dessus,
en présence d'un diluant et, le cas échéant, en présence d'une base,
et en outre, le cas échéant,
d) on transforme les composés hydroxyliques obtenus selon les procédés aJ, b) ou c) en présence d'un diluant en l'alcoolate de métal alcalin et on fait réagir ce dernier avec un halogénure pour former les éthers correspondants, puis on additionne encore, éventuellement, un acide ou un sel métallique sur les composés de formule (I) ainsi obtenus.

4. Compositions fongicides, caractérisées par une teneur en au moins un dérivé hydroxyalkylazolylique à substituant hétérocyclique de formule (I) suivant la revendication 1 ou un sel d'addition d'acide ou un complexe de sel métallique d'un composé de formule (I).

5. Utilisation de dérivés hydroxyalkylazolyliques à substituant hétérocyclique de formule (I) suivant la revendication 1 ou de sels d'addition d'acides ou de complexes de sels métalliques de ces dérivés comme agents phytosanitaires.

6. Utilisation de dérivés hydroxyalkylazolyliques à substituant hétérocyclique de formule (I) suivant la revendication 1 et de leurs sels d'addition d'acides ou de leurs complexes de sels métalliques pour combattre des champignons.

7. Procédé de préparation de compositions fongicides, caractérisé en ce qu'on mélange des dérivés hydroxyalkylazolyliques à substituant hétérocyclique de formule (I) suivant la revendication 1 ou leurs sels d'addition d'acides ou leurs complexes de sels métalliques avec des diluants et/ou des agents tensio-actifs.
